# FASCICULE DE BREVET EUROPEEN

(11) **EP 2 297 095 B1**
(45) Date de publication et mention de la délivrance du brevet: **25.04.2012**
(21) Numéro de dépôt: 09745768.3
(22) Date de dépôt: 13.05.2009
(51) Int. Cl.: C07C 323/60, C07C 323/65

(54) **NOUVEAUX DÉRIVÉS D'AMINO-ACIDES, LEUR PROCÉDÉ DE PRÉPARATION ET LEUR UTILISATION THÉRAPEUTIQUE**
NEUE AMINOSÄUREDERIVATE, VERFAHREN ZU DEREN HERSTELLUNG UND DEREN THERAPEUTISCHE VERWENDUNG
NOVEL AMINO ACID DERIVATIVES, METHOD FOR PREPARING SAME, AND THERAPEUTIC USE THEREOF

(30) Priorité: 13.05.2008 FR 0853092
(43) Date de publication de la demande: 23.03.2011
(73) Titulaire: Pharmaleads, 75013 Paris (FR)
(72) Inventeur: FOURNIE-ZALUSKI, Marie-Claude, F-75011 Paris (FR); PORAS, Hervé, F-78870 Bailly (FR); ROQUES, Bernard, F-75014 Paris (FR)
(74) Mandataire: Warcoin, Jacques
(86) Numéro de dépôt international: PCT/EP2009/055787
(87) Numéro de publication internationale: WO 2009/138436

(56) Documents cités:
- WO-A-2007/048787
- US-A- 4 426 391
- ALEXANDER J ET AL: "(ACYLOXY)ALKYL CARBAMATES AS NOVEL BIOREVERSIBLE PRODRUGS FOR AMINES: INCREASED PERMEATION THROUGH BIOLOGICAL MEMBRANES" JOURNAL OF MEDICINAL CHEMISTRY, US AMERICAN CHEMICAL SOCIETY. WASHINGTON, vol. 31, no. 2, 1 janvier 1988 (1988-01-01), pages 318-322, XP000651965 ISSN: 0022-2623

## Description

L'invention concerne de nouveaux inhibiteurs mixtes de la néprilysine et de l'aminopeptidase N à action prolongée.

On sait que les enképhalines -Tyr-Gly-Gly-Phe-Met et Tyr-Gly-Gly-Phe-Leu-sont les ligands endogènes des récepteurs opioïdes µ et δ, dont les localisations (Waksman et al. (1985) Proc. Natl. Acad. Sci. USA, 83, 1523-1527) et les fonctions sont différentes : les récepteurs µ sont essentiellement impliqués dans la transmission des influx nociceptifs et les récepteurs δ dans la régulation de l'humeur et les comportements d'adaptation, en particulier au stress (revue dans Noble et Roques, 2007, Expert Opin. Ther. Targets, 11, 145-159), (Jutkiewicz et al., 2006, Eur. J. Pharmacol, 531, 151-159).

L'administration intracérébroventriculaire d'enképhalines exogènes induit une réponse analgésique fugace à cause du catabolisme très rapide de ces peptides par deux enzymes, la néprilysine (NEP, E.C. 3.4.24.11) qui clive la liaison Gly³-Phe⁴ des enképhalines et l'aminopeptidase N (APN, E.C.3.4.11.2) qui libère la Tyrosine N-terminale (revue dans Roques et al. 1993, Pharmacol. Rev. 45, 88-146).

On connaît des inhibiteurs mixtes de ces deux enzymes, qui, en protégeant complètement les enképhalines endogènes de leur dégradation enzymatique, révèlent les activités pharmacologiques, en particulier analgésiques et antidépressives, des enképhalines. Les inhibiteurs mixtes de ces deux activités enzymatiques, décrits dans l'art antérieur, sont des composés à fonction hydroxamate (FR 2 518 088 et FR 2 605 004), des composés aminophosphiniques (FR 2 755 135 et FR 2 777 780) et des dérivés d'aminoacides (FR 2 651 229 et WO2007/048787). Dans le cas des composés à fonction hydroxamate, une bonne activité in vitro et in vivo après administration par voie intracérébroventriculaire a été observée (Eur. J. Pharmacol., 102, (1984), 525-528 ; Eur.J.Pharmacol., 165, (1989), 199-207 ; Eur.J.Pharmacol.,192, (1991), 253-262) ; une activité significative a pu également être démontrée après administration intraveineuse (iv) dans un modèle de rat arthritique (Brain Research, 497, (1989), 94-101). Dans le cas des dérivés phosphiniques et des dérivés d'aminoacides décrits dans la demande FR 2 651 229, une bonne activité in vivo a été démontrée après administration par voie iv, lorsque les molécules étudiées ont été solubilisées dans un mélange d'huile, d'éthanol et d'eau (J.Med.Chem., 43, (2000), 1398-1408 ; J.Med Chem., 44, (2001), 3523-3530 ; J.Pharm.Exp.Ther., 261, (1992), 181-190). Les dérivés d'aminoacides décrits dans la demande W02007/048787 sont des inhibiteurs mixtes, solubles en milieu aqueux, qui présentent des propriétés analgésiques après administration par voie iv et par voie orale chez l'animal de laboratoire, à des doses compatibles avec une administration chez l'homme. Malheureusement, ces molécules présentent, dans les modèles animaux de douleurs, une durée d'action courte (environ 40 min) avec un maximum autour de 10 min. et un retour aux conditions normales après 15-30 min. qui peut représenter un handicap important pour des utilisations thérapeutiques, si la durée d'action était du même ordre chez l'homme.

On rappellera que la durée d'action est le temps pendant lequel, à son site d'action, le principe actif contenu dans un médicament produit son effet thérapeutique ou préventif Il est ensuite éliminé par l'organisme.

Dans le but d'améliorer la durée d'action de ces molécules des modifications dans leurs structures ont été apportées.

L'un des objets de l'invention est de fournir de nouveaux composés hydrosolubles capables d'inhiber conjointement les deux activités enzymatiques responsables de la dégradation des enképhalines et de manifester leurs propriétés pharmacologiques sur des tests centraux et périphériques après administration, notamment par voie iv ou par voie orale, et dont la durée d'action sur l'animal de laboratoire est égale ou supérieure à 120 min.

De ce fait, les nouveaux composés présentent des propriétés des substances morphiniques, en particulier l'analgésie, les effets bénéfiques sur le comportement (diminution de la composante émotionnelle de la douleur et réponses antidépressives) et des effets périphériques (antidiarréique, antitussique, anti-inflammatoire) sans en avoir les inconvénients majeurs (tolérance, dépendances physique et psychique, dépression respiratoire, constipation, nausée, etc..).

De plus les douleurs inflammatoires, neurogéniques et neuropathiques, dont la composante périphérique est importante, et les douleurs nociceptives sont réduites voire éliminées par les composés de l'invention administrés, par voie orale notamment, et ce, sans que ceux-ci ne soient contraints d'atteindre le système nerveux central. Ce résultat, très intéressant mais inattendu, a été formellement démontré par utilisation d'un antagoniste - le methylnaloxonium- incapable de rentrer dans le cerveau (Milne R.J. et al. (1990) Neuroscience Lett. 114,259-264). Ceci réduit totalement tous les effets dus à la stimulation des récepteurs opioïdes cérébraux par les composés de l'invention, sans altérer les effets analgésiques des composés sur ces douleurs, en particulier neurogénique, neuropathique, neuroinflammatoire et nociceptives.

Un autre objet de l'invention est de proposer des associations entre des composés connus pour leurs propriétés antinociceptives mais présentant à fortes doses des effets secondaires néfastes, et les composés revendiqués dans la présente invention. Ces associations concernent plus particulièrement la morphine et ses dérivés, le Δ⁹ tetrahydrocannabinol (Δ⁹ THC) et ses dérivés ainsi que les dérivés du Gaba tels que la gabapentine ou la prégabaline. On a en effet pu constater une forte potentialisation des réponses antinociceptives obtenues par combinaison de doses subactives d'un des composés revendiqués dans la présente demande, et d'un des analgésiques précédemment cités (morphine, Δ⁹ THC, Gabapentine).

L'invention a plus particulièrement pour objet des composés répondant à la formule (I) suivante :

R₁NH-CH(R₂)-CH₂-S-S-CH₂-C(R₃)(R₄)-CONH-C(R₅)(R₆)-COOR₇

dans laquelle :
R₁ représente un groupement (acyloxy)alkyl carbamate -C(O)-O-C(R₈)(R₉)-OC(O)-R₁₀, dans lequel
   - R₈ et R₉ représentent indépendamment l'un de l'autre un hydrogène, un groupement alkyle, aryle, arylalkyle, cycloalkyle, cyclohétéroalkyle, hétéroalkyle, hétéroaryle ou hétéroaryalkyle ; ou
   - pris ensemble, R₈ et R₉ peuvent former un cycloalkyle à 5 ou 6 chaînons ;
   - R₁₀ représente un groupement alkyle, aryle, arylalkyle, cycloalkyle, cyclohétéroalkyle, hétéroalkyle, hétéroaryle ou hétéroaryalkyle ;
R₂ représente :
   - une chaîne hydrocarbonée saturée (alkyle) linéaire ou ramifiée comportant de 1 à 6 atomes de carbone, éventuellement substituée par :
      * un radical OH, OR₁₁, SH, SR₁₁ ou S(O)R₁₁, dans chacun de ces radicaux R₁₁ représente une chaîne hydrocarbonée linéaire ou ramifiée de 1 à 4 atomes de carbone, un radical phényle ou un radical benzyle,
      * un radical phényle ou benzyle, éventuellement substitué par :
         - 1 à 5 atomes d'halogène, notamment le fluor,
         - un radical OH, OR₁₁, SH, SR₁₁, S(O)R₁₁, R₁₁ ayant la même signification que précédemment,
   - un radical méthylène substitué par un hétérocycle à 5 ou 6 chaînons, aromatique ou saturé, possédant comme hétéroatome, un atome d'azote ou de soufre, éventuellement oxydé sous forme N-oxyde ou S-oxyde,
lorsque R₄ représente un atome d'hydrogène, R₃ représente :
   - un radical phényle ou benzyle éventuellement substitué par :
      * 1 à 5 atomes d'halogène ;
      * un radical SR₁₁, S(O)R₁₁, ou OR₁₁, R₁₁ ayant la même signification que précédemment ;
      * un groupement amino éventuellement mono- ou disubstitué par un groupement aliphatique, cyclique ou linéaire, de 1 à 6 atomes de carbone ;
   - un hétéroaryle à 5 ou 6 chaînons, l'hétéroatome étant un oxygène, un soufre ou un azote ;
   - un groupe méthylène substitué par un hétérocycle à 5 ou 6 chaînons, aromatique ou saturé, l'hétéroatome étant un oxygène, un azote ou un soufre, les atomes d'azote et de soufre pouvant être oxydés sous forme de N-oxyde ou de S-oxyde ;
lorsque R₄ est différent de H, R₁ et R₄ pris ensemble forment un cycle saturé à 5 ou 6 chaînons ;
R₅ et R₆ représentent indépendamment l'un de l'autre :
   - un hydrogène,
   - une chaîne hydrocarbonée saturée (alkyle), linéaire ou ramifiée, comportant de 1 à 6 atomes de carbone, éventuellement substitué par un radical OH, OR₁₁, SH ou SR₁₁,COOH ou COOR₁₁ dans chacun de ces radicaux R₁₁ a la même signification que précédemment,
   - un radical phényle ou benzyle, éventuellement substitué par :
      * une chaîne alkyle, linéaire ou ramifiée, de 1 à 4 atomes de carbone ;
      * 1 à 5 halogènes, notamment le fluor ou le brome ;
      * un radical OH, OR₁₁ ou SR₁₁, R₁₁ ayant la même définition que précédemment ;
ou pris ensemble R₃ et R₆ forment un cycle saturé à 5 ou 6 chaînons ;
R₇ représente
   - un hydrogène ;
   - un radical phényle ou benzyle éventuellement substitué par 1 à 5 halogènes, notamment le fluor ;
   - un groupement de formule CR₁₂(R₁₃)C(O)OR₁₄ ;
   - un groupement CR₁₂(R₁₃)OC(O)R₁₄;
   - un groupement CR₁₂(R₁₃)OC(O)OR₁₄;
R₁₂ et R₁₃ représentent indépendamment l'un de l'autre un hydrogène, un groupement alkyle, aryle, arylalkyle, cycloalkyle, cyclohétéroalkyle, hétéroalkyle, hétéroaryle ou hétéroaryalkyle ;
pris ensemble R₁₂ et R₁₃ peuvent constituer un cycloalkyle à 5 ou 6 chaînons.
R₁₄ représente un groupement alkyle, aryle, arylalkyle, cycloalkyle, cyclohétéroalkyle, hétéroalkyle, hétéroaryle ou hétéroaryalkyle ;
ainsi que les sels d'addition dudit composé (I) avec des bases minérales ou organiques pharmaceutiquement acceptables et chacun de ses isomères, en particulier ses isomères optiques (énantiomères et diastéréoisomères).

Pour la partie « drogue » de la molécule, à savoir la partie de formule -NH-C*H(R₂)-CH₂-S-S-CH₂-C*(R₃)(R₄)-CONH-C*(R₅)(R₆)-COO-, les composés selon l'invention possèdent potentiellement au maximum 3 carbones asymétriques, signalés par un astérisque, et qui se réduit à un seul centre d'asymétrie lorsque (R₃)(R₄) et (R₅) (R₆) forment des cycles dépourvus d'asymétrie. Ces centres sont optiquement purs, de configuration absolue analogue à celle d'un aminoacide naturel, c'est-à-dire de configuration S. Les centres d'asymétrie éventuels des parties « prodrogues », c'est-à-dire pour les substituants R₁ et R₇, ne sont pas résolus : ces centres de symétries potentiels peuvent donc être, d'une manière indifférente, de configuration R ou S.

L'invention a également pour objet les sels d'addition des composés de formule (I), obtenus avec des bases organiques ou minérales pharmacologiquement acceptables.

Dans la présente invention, on entend désigner par "pharmaceutiquement acceptable" ce qui est utile dans la préparation d'une composition pharmaceutique, qui est généralement sûr, non toxique et ni biologiquement ni autrement non souhaitable et qui est acceptable pour une utilisation vétérinaire de même que pharmaceutique humaine.

En outre, on entend désigner par "sels pharmaceutiquement acceptables" d'un composé des sels qui possèdent l'activité pharmacologique souhaitée du composé parent. De tels sels comprennent les sels formés lorsqu'un proton acide présent dans le composé parent soit est remplacé par un ion métallique, par exemple un ion de métal alcalin, un ion de métal alcalino-terreux ; soit se coordonne avec une base organique ou inorganique. Les bases organiques acceptables comprennent la diéthanolamine, l'éthanolamine, N-méthylglucamine, la triéthanolamine, la trométhamine et similaires ou des acides aminés basiques naturels (par exemple lysine, arginine, alanine, asparagine, acide aspartique, cystéine, glutamine, acide glutamique, glycine, histidine, isoleucine, leucine, méthionine, phénylalanine, proline, sérine, thréonine, tryptophane, tyrosine et valine) ou non naturels (telle que la pseudo-lysine). Les bases inorganiques acceptables comprennent en particulier l'hydroxyde d'aluminium, l'hydroxyde de calcium, l'hydroxyde de potassium, l'hydroxyde de lithium, le carbonate de sodium et l'hydroxyde de sodium. Avantageusement, le proton acide est déplacé par un ion Na⁺, notamment en utilisant de l'hydroxyde de sodium.

Dans le cadre de la présente invention, l'expression « chaîne hydrocarbonée » désigne des alcanes, des alcènes ou des alcynes, linéaires ou ramifiés. En particulier, l'expression « chaîne hydrocarbonée saturée » désigne des radicaux alkyles comportant de 1 à 6 atomes de carbone (C₁-C₆) ou de 1 à 4 atomes de carbone (C₁-C₄), linéaires ou ramifiés. Comme exemple de radicaux alkyles comportant de 1 à 4 atomes de carbones, on peut citer les radicaux méthyle, éthyle, propyle, butyle, isopropyle, 1-méthyl-éthyle, 1-méthyl-propyle, 2-méthyl-propyle. Comme exemple de radicaux alkyles comportant de 1 à 6 atomes de carbones, on peut en outre citer les radicaux pentyle, hexyle, 1-méthyl-butyle, 1-méthyl-pentyle, 2-méthyl-butyle, 2-méthyl-pentyle, 3-méthyl-butyle, 3-méthyl-pentyle, 4-méthyl-pentyle ou 1-éthyl-propyle, 1-éthyl-butyle, 2-éthyl-butyle. L'expression «chaîne hydrocarbonée insaturée » désigne des radicaux alcényle (au moins une double liaison), par exemple vinyle, allyle ou analogue, ou alcynyle (au moins une triple liaison) comportant de 2 à 6 atomes de carbone, ou de 2 à 4 atomes de carbone, linéaires ou ramifiés.

Par le terme "hétéroalkyle", on entend au sens de la présente invention toute chaîne hydrocarbonée, telle que définie précédemment, contenant un ou plusieurs hétéroatomes, tels que par exemple des atomes de soufre, d'azote ou d'oxygène.

Par le terme "cycloalkyle", on entend au sens de la présente invention tout cycle hydrocarboné, saturé ou non, mais non aromatique, de 3 à 7 chaînons, en particulier de 5 ou 6 chaînons, tels que le cyclopentyle et le cyclohexyle.

Par le terme "cyclohétéroalkyle", on entend au sens de la présente invention tout cycle hydrocarboné, saturé ou non, mais non aromatique, de 5 à 7 chaînons, contenant un ou plusieurs hétéroatomes, tels que par exemple des atomes de soufre, d'azote ou d'oxygène.

Par l'expression « groupement aliphatique, cyclique ou linéaire », on entend une « chaîne hydrocarbonée » ou un "cycloalkyle" tels que définis précédemment.

Par le terme "aryle", on entend au sens de la présente invention un ou plusieurs cycles aromatiques ayant de 5 à 10 atomes de carbone, pouvant être accolés. En particulier, les groupes aryles peuvent être des groupes monocycliques ou bicycliques, comme par exemple le groupe phényle ou naphthyle. Avantageusement, le groupe aryle est un phényle.

Par le terme "hétéroaryle", on entend au sens de la présente invention tout groupe aromatique comprenant de 5 à 10 atomes cycliques, qui sont des atomes de carbone et un ou plusieurs hétéroatomes, tels que par exemple des atomes de soufre, d'azote ou d'oxygène. L'hétéroaryle selon la présente invention peut être constitué par un ou deux cycles accolés. Des exemples de groupes hétéroaryles sont les groupes quinolyle, isoquinolyle, imidazolyle, indolyle, pyridyle, triazinyle, thiazoyle et thiophényle.

Le terme « aralkyle » dans le cadre de la présente invention désigne des radicaux aryles (tels que définis précédemment) liés à des radicaux alkyles (tels que définis précédemment), comme par exemple le benzyle ou le phénéthyle.

Le terme « hétéroaralkyle » dans le cadre de la présente invention désigne des radicaux hétéroaryles (tels que définis précédemment) liés à des radicaux alkyles (tels que définis précédemment).

Par le terme « hétérocycle », on entend un "cyclohétéroalkyle" ou un "hétéroaryle" tels que définis précédemment. Comme exemple de noyaux hétérocycliques à 5 ou 6 atomes, aromatiques ou saturés, possédant comme hétéroatome un atome d'azote ou de soufre, on peut citer, mais sans limitation, les radicaux suivants : thiényle, pyrrolyle, imidazolyle, pyrazolyle, isothiazolyle, pyridyle, pyrazinyle, pyrimidinyle, pyridazinyle, pyrrolidinyle, pyrrolinyle, imidazolidinyle, pyrazolidinyle, pyrazolinyle, piperidyle, piperazinyle, thiadiazolyle, les atomes d'azote et de soufre étant éventuellement oxydés sous forme de N-oxyde ou de S-oxyde. Comme exemple de noyaux hétérocycliques à 5 ou 6 atomes, aromatiques ou saturés, possédant comme hétéroatome un atome d'oxygène, on peut citer, mais sans limitation, les radicaux suivants : furyle, pyranyle, isoxazolyle, morpholinyle, furazanyle, oxazolyle, oxazolidinyle, oxazolinyle.

Le terme « halogène » utilisé ici désigne un chlore, un brome, un iode et un fluor.

Le radical R₁ représente avantageusement un groupement (acyloxy)alkyl carbamate -C(O)-O-C(R₈)(R₉)-OC(O)-R₁₀, dans lequel
- R₈ et R₉ représentent indépendamment l'un de l'autre un hydrogène ou un groupement alkyle ; et
- R₁₀ représente un groupement alkyle, en particulier un isopropyle.

Le radical R₂ représente avantageusement un radical alkyle ayant de 1 à 4 atomes de carbone, éventuellement substitué par un radical OR₁₁, SR₁₁ ou S(O)R₁₁, dans chacun de ces radicaux R₁₁ a la même signification que précédemment. R₂ représente encore plus avantageusement un radical alkyle ayant de 1 à 4 atomes de carbone substitué par un radical SR₁₁ ou S(O)R₁₁, R₁₁ ayant la même signification que précédemment, en particulier R₁₁ représente une chaîne hydrocarbonée saturée linéaire ou ramifiée de 1 à 4 atomes de carbone et plus avantageusement un groupe méthyle.

Selon une variante avantageuse de l'invention, le radical R₄ représente un atome d'hydrogène. Dans le cadre de cette variante, le radical R₃ représente avantageusement :
- un radical benzyle ou phényle,
- un radical méthylène substitué par un hétérocycle à 5 ou 6 atomes, aromatique ou saturé, possédant comme hétéroatome, un atome d'azote ou de soufre, éventuellement oxydé sous forme de N-oxyde ou de S-oxyde.

En particulier, le radical R₄ représente un atome d'hydrogène et le radical R₃ représente un radical benzyle ou un radical méthylène substitué par un hétérocycle à 5 ou 6 atomes, aromatique ou saturé, possédant comme hétéroatome, un atome d'azote ou de soufre, éventuellement oxydé sous forme de N-oxyde ou de S-oxyde, encore plus avantageusement un radical benzyle.

Selon une autre variante avantageuse de l'invention, les radicaux R₄ et R₃ forment conjointement, avec l'atome de carbone qui les porte un cycloalkyle à 5 ou 6 chaînons, en particulier un cyclopentane ou un cyclohexane.

Le radical R₅ représente avantageusement un atome d'hydrogène.

Le radical R₆ représente avantageusement un atome d'hydrogène ou un radical alkyle ayant de 1 à 6 atomes de carbone, encore plus avantageusement 1 à 4 atomes de carbone, éventuellement substitué par un radical OH, OR₁₁, SH ou SR₁₁,COOH ou COOR₁₁ dans chacun de ces radicaux R₁₁ ayant la même signification que précédemment. Le radical R₆ représente encore plus avantageusement un atome d'hydrogène ou un radical alkyle ayant de 1 à 6 atomes de carbone, encore plus avantageusement 1 à 4 atomes de carbone, substitué par un radical OH, SH, COOH ou COOR₁₁, R₁₁ ayant la même signification que précédemment.

Le radical R₇ représente avantageusement :
- un atome d'hydrogène ;
- un radical phényle ou benzyle ;
- un radical alkyle ayant de 1 à 4 atomes de carbone ;
- un groupement -CR₁₂(R₁₃)O(CO)OR₁₄, dans lequel R₁₂, R₁₃ et R₁₄ ont la même signification que précédemment, en particulier R₁₂ représente un hydrogène et R₁₃ et R₁₄ représentent, indépendamment l'un de l'autre, un groupe alkyle en C₁-C₄ éventuellement substitué par un groupe méthoxy ou un groupe cycloalkyle en C₅-C₆.

En particulier, le radical R₇ représente un atome d'hydrogène ou un radical benzyle.

L'invention concerne en particulier les composés suivants :
Ester 1-(1-{2-[(1-éthoxycarbonyloxy-éthoxycarbonylméthyl)-carbamoyl]-3-phényl-propyldisulfanylméthyl }-3-méthylsulfanyl-propylcarbamoyloxy)-éthyl de l'acide isobutyrique.
Ester 1-{1-[2-(benzyloxycarbonylméthyl-carbamoyl)-3-phényl-propyldisulfanylméthyl] -3-méthylsulfanyl-propylcarbamoyloxy}-éthyl de l'acide isobutyrique
Ester 1-{1-[2-(carboxymétyl-carbamoyl)-3-phényl-propyldisulfanylméthyl]-3-méthylsulfanyl-propylcarbamoyloxy}-éthyl de l'acide isobutyrique
Ester 1-(1-{2-[(1-éthoxycarbonyloxy-éthoxycarbonylméthyl)-carbamoyl]-3-phényl-propyldisulfanylméthyl}-3-méthanesulfinyl-propylcarbamoloxy)-éthyl de l'acide isobutyrique
Ester 1-{1-[2-benzyloxycarbonylméthyl-carbamoyl)-3-phényl-propyldisulfanylméthyl]-3-méthanesulfinyl-propylcarbamoyloxy}-éthyl de l'acide isobutyrique
Ester 1-{1-2[2-(carboxyméthyl-carbamoyl)-3-phényl-propyldisulfanylméthyl]-3-méthanesulfinyl-propylcarbamoyloxy}-éthyl de l'acide isobutyrique
Acide 2-({1-[2-(1-Isobutyryloxy-ethoxycarbonylamino)-4-methylsulfinyl-butyldisulfanylmethyl]-cyclopentanecarbonyl}-amino)-succinique
Acide 2-({1-[2-(1-Isobutyryloxy-ethoxycarbonylamino)-4-methylsulfanyl-butyldisulfanylmethyl]-cyclopentanecarbonyl}-amino)-succinique
Ester benzylique de l'acide 2-({1-[2-(1-Isobutyryloxy-ethoxycarbonylamino)-4-methanesulfinyl-butyldisulfanylmethyl]-cyclopentanecarbonyl}-amino)-succinique
Ester benzylique de l'acide 2-({1-[2-(1-Isobutyryloxy-ethoxycarbonylamino)-4-methylsulfanyl-butyldisulfanylmethyl]-cyclopentanecarbonyl}-amino)-succinique
Selon une variante avantageuse de l'invention, les composés suivants sont préférés :
Ester 1-{1-[2-(carboxymétyl-carbamoyl)-3-phényl-propyldisulfanylméthyl]-3-méthylsulfanyl-propylcarbamoyloxy}-éthyl de l'acide isobutyrique
Ester 1-{1-[2-benzyloxycarbonylméthyl-carbamoyl)-3-phényl-propyldisulfanylméthyl]-3-méthanesulfinyl-propylcarbamoyloxy}-éthyl de l'acide isobutyrique
Ester 1-{1-2[2-(carboxyméthyl-carbamoyl)-3-phényl-propyldisulfanylméthyl]-3-méthanesulfinyl-propylcarbamoyloxy}-éthyl de l'acide isobutyrique
Acide 2-({1-[2-(1-Isobutyryloxy-ethoxycarbonylamino)-4-methanesulfinyl-butyldisulfanylmethyl]-cyclopentanecarbonyl}-amino)-succinique
Acide 2-({1-[2-(1-Isobutyryloxy-ethoxycarbonylamino)-4-methylsulfanyl-butyldisulfanylmethyl]-cyclopentanecarbonyl}-amino)-succinique
Ester benzylique de l'acide 2-({1-[2-(1-Isobutyryloxy-ethoxycarbonylamino)-4-methanesulfinyl-butyldisulfanylmethyl]-cyclopentanecarbonyl}-amino)-succinique
Ester benzylique de l'acide 2-({1-[2-(1-Isobutyryloxy-ethoxycarbonylamino)-4-methylsulfanyl-butyldisulfanylmethyl]-cyclopentanecarbonyl}-amino)-succinique

Les composés de formules (I) sont obtenus :
- par condensation d'un β-aminothiol protégé sur la fonction amine par un groupement ter-butyloxycarbonyl (Boc) (II) avec un acide mercaptoalkanoïque (III) au moyen du méthoxycarbonylsulfenylchloride.

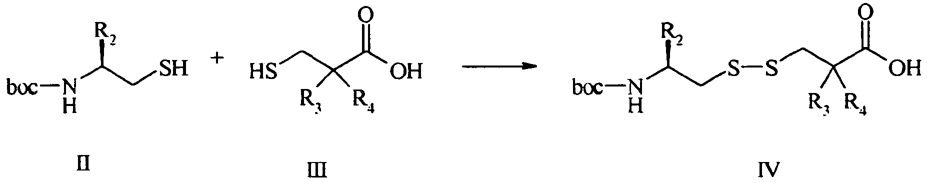

Le disulfure IV ainsi obtenu est couplé dans les conditions classiques du couplage peptidique, de préférence par action du TBTU (O-(Benzotriazol-1-yl)-N,N,N',N'-tetramethyluronium tetrafluoroborate) en présence de DIEA (N,N-diisopropyléthylamine), avec un aminoester V, pour conduire au composé VI.

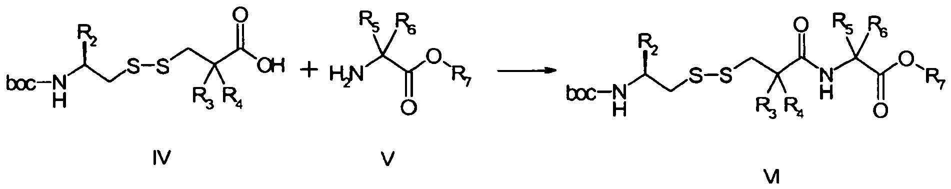

La déprotection du groupement Boc du composé VI est effectué par action de l'acide formique et le composé VII ainsi formé réagit sur un ester activé du carbamate VIII (R₁-O-(p.NO₂)Ph ou R₁-O-succinimide) pour conduire au composé de formule (I).

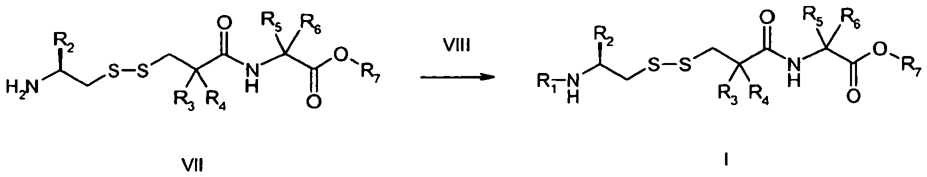

Le Boc-β-aminothiol (II) est obtenu en trois étapes à partir du Boc-α-aminoacide commercial correspondant, de configuration absolue S, avec rétention de configuration, selon un procédé bien connu de l'homme de l'art (J. Med. Chem., 35, 1992, 1259).

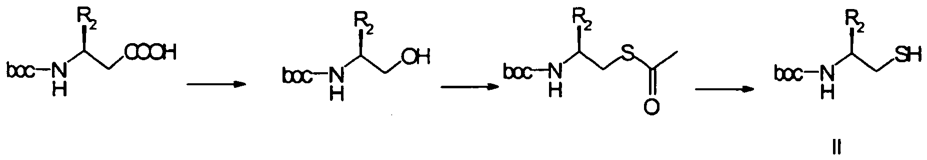

Deux procédés différents sont utilisés pour synthétiser l'acide mercapto alkanoïque (III) en fonction de la nature des groupements R₃ et R₄.
-Si R₄=H, le composé (III) est obtenu à partir de l'acide malonique correspondant, qui selon un procédé bien connu de l'homme de l'art (Ber. 57, (1924), 1116) est transformé en acrylate (IX). L'addition d'acide thioacétique, à l'acrylate (IX) conduit au dérivé (X) racémique, qui est estérifié, par exemple, par le méthanol MeOH en présence d'EDCl (1-(3-diméthylaminopropyl)-3-éthylcarboiimide) et DMAP (4-diméthylaminopyridine) ou par le méthanol en présence de chlorure de thionyle. Une résolution par l'α-chymotrypsine permet d'isoler le thioacétate de configuration S (XII) (Bioorg. Med. Chem. Lett., 3, 1993, 2681). L'hydrolyse alcaline du thioester conduit au composé (III). 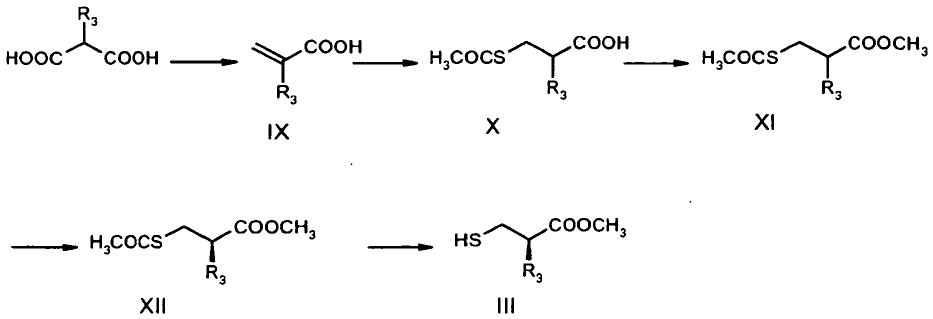 - Si R4 # de H, le composé (III) est obtenu à partir de l'acide carboxylique correspondant (XIII). Celui-ci, traité par le chloroformiate d'éthyle en présence de LDA (Lithium diisopropyl amide) dans le THF (tétrahydrofurane), conduit au composé (XIV). La fonction acide carboxylique de (XIV) est transformée en anhydride mixte et est réduite par NaBH₄ en alcool (XV). L'activation de l'alcool en mésylate, puis la substitution par le thioacétate de potassium conduit à (XVI), qui par hydrolyse alcaline donne (III).

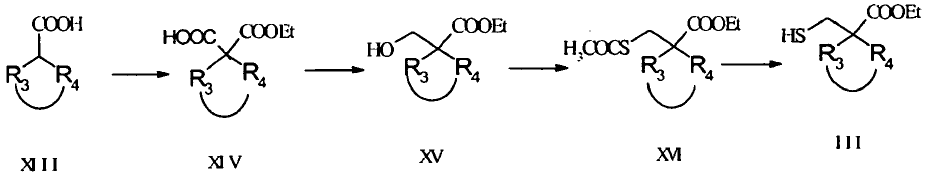

Une autre voie de synthèse du composé III peut être proposée à partir de l'acide XIII. Celui-ci est transformé en ester t-butylique XVII, et par traitement au LDA dans le THF puis carbonatation par le CO₂ conduit au dérivé XVIII. La fonction acide de XVIII est alors réduite en alcool pour conduire au composé XIX. La suite des réactions est identique à celle proposée dans la voie de synthèse précédente.

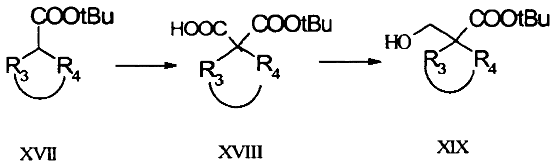

Un autre objet de l'invention est l'utilisation à titre de médicament des composés tels que définis précédemment ou obtenus par un procédé tel que défini précédemment. L'invention a aussi pour objet les compositions pharmaceutiques contenant à titre de principe actif au moins un des composés de formule générale (I) ou un de ses sels ou hydrates de ses sels en combinaison avec un ou plusieurs support inertes ou autres véhicules pharmaceutiquement acceptables.

Ces composés présentent les propriétés des substances morphiniques, en particulier l'analgésie, et en particulier dans ses composantes périphériques (inflammatoires, neurogéniques et neuropathiques), les effets bénéfiques sur le comportement, en particulier en cas de dépression et/ou d'anxiété, sans en avoir les inconvénients majeurs (tolérance, dépendance, dépression respiratoire, constipation, etc..). Ainsi à l'encontre des agonistes opioides exogènes interagissant avec les récepteurs delta, les inhibiteurs mixtes selon l'invention ont des effets antidépresseurs sans provoquer de risque de déclenchement de crise épileptiformes ou de convulsions et agissent rapidement (Baamonde A. et al. 1992, Jutkiewicz E.M. et al., 2005). Ces composés agissent à la périphérie au niveau des nocicepteurs (Stein C. et al. (1993) Lancet 342 321-324 (2003) Nature Med., 9, 119-124). D'une manière avantageuse, les composés selon l'invention, administrés par voie orale, ne pénètrent pas dans le système nerveux central en concentrations importantes : ceci est confirmé par l'observation que la pré-administration d'un antagoniste - le méthylnaloxonium- incapable de franchir la barrière hématoméningée bloque l'action analgésique des composés selon l'invention.

La principale application des composés selon l'invention est donc dans le domaine de l'analgésie, des antidépresseurs et du traitement des addictions. Ces compositions peuvent être utilisées en particulier comme analgésique puissant dans les douleurs neuroinflammatoires, neurogéniques, neuropathiques et nociceptives, et comme anti-dépresseur. En outre, les composés selon l'invention, de formule (I) ont montré après administration par voie orale des effets tout à fait intéressants sur des modèles animaux prédictifs des activités chez l'homme, dans :
- les douleurs neuropathiques diverses, neuropathie diabétique, neuropathie déclenchée par pré-administration d'un anticancéreux, d'un antiviral (VIH-1), zona etc.. ;
- l'hyperalgésie et l'allodynie : hyperalgésie et allodynie neurophatique et neuroinflammatoire, douleur provoquée par l'administration de formaline, de carragénine, d'adjuvant de Freund, hyperalgésie et allodynie produites par compression partielle et unilatérale du nerf sciatique, par administration de cellules tumorales dans la moelle osseuse etc

Par médicaments analgésiques on entendra des médicaments qui soulagent ou suppriment la douleur sans entraîner la perte de sensations ou de la conscience.

En résumé, la présente invention vise le traitement des symptômes correspondant non seulement à des douleurs par excès de stimulations nociceptives, mais encore des douleurs neuropathiques ou neurogéniques qui n'ont plus un rôle physiologique, par exemple sous la forme d'un signal, mais sont devenues réellement pathologiques et chroniques.

Parmi les douleurs chroniques neuropathiques et neurogéniques potentiellement sensibles à l'action des composés de formule (I), on peut citer, à titre d'exemples non limitatifs, les douleurs des neuropathies périphériques ou centrales résultant de lésions nerveuses d'origine traumatique (e.g plexus brachial), métabolique (e.g., diabète, neuropathie alcoolique), infectieuse (e.g., zona, herpes), toxique (e.g., arsenic, plomb), invasive (douleur cancéreuse) ou congénitale, radiculopathiques (e.g. dorsolombaire ou cervicale), névralgiques (trijumeau) ; les douleurs des membres fantômes ; les douleurs articulaires non inflammatoires (e.g., arthrose) ; les fibromyalgies ; les douleurs rachidiennes ; les douleurs post-opératoires ; les douleurs médicamenteuses (e.g. antitumoraux, antiviraux).

Les composés selon l'invention peuvent également être utilisés dans le traitement de la sclérose en plaque, qui est une maladie inflammatoire du système nerveux central.

D'une manière très intéressante, les composés selon l'invention ont une durée d'action longue, en particulier égale ou supérieure à 120 minutes, plus avantageusement égale ou supérieure à 150 minutes, encore plus avantageusement égale ou supérieure à 180 minutes.

Les compositions pharmaceutiques selon l'invention peuvent être, à titre d'exemple, des compositions administrables par voie orale, nasale (administration par aérosol), sublinguale (administration par diffusion perlinguable), rectale, parentérale intraveineuse et percutanée. A titre d'exemple de compositions administrables par voie orale, on peut citer les comprimés, les gélules, les granules, les microsphères, les poudres et les solutions ou suspensions orales.

D'une manière très intéressante également, les composés selon l'invention se sont révélés particulièrement appropriés pour une administration par voie orale.

Cette voie d'administration permet ainsi une action de la composition selon l'invention sans pénétrer dans le système nerveux central. Ceci est particulièrement intéressant pour éliminer tous les effets non désirés résultant de l'activation des récepteurs opoïdes dans le cerveau et/ou la moelle épinière. Il en est de même lorsque la composition comprend des composés complémentaires, qui peuvent présenter des effets non désirés sur le système nerveux central tels que par exemple des cannabinoïdes naturels ou des dérivés de synthèse. Ceci permet également d'augmenter la biodisponibilité cérébrale des composants des associations.

Selon une variante avantageuse de l'invention, les composés de formule (I) sont utilisés en association avec des cannabinoïdes.

Au sens de la présente invention, on entend par l'expression « cannabinoïdes » le Δ⁹ THC, des agonistes du récepteur CB1 synthétiques ou des inhibiteurs de la dégradation de l'anandamide. Les cannabinoïdes introduits dans les compositions selon l'invention sont de préférence le Δ⁹ THC.

L'invention a également pour objet l'association des nouveaux composés selon l'invention avec la morphine ou l'un de ses dérivés.

L'invention a également pour objet, plus particulièrement, l'association des nouveaux composés selon l'invention avec les dérivés du Gaba, tels que la gabapentine ou la prégabaline.

L'invention a également pour objet une composition pharmaceutique comprenant au moins un composé de formule (I) tel que défini précédemment, au moins :
- un dérivé de cannabinoïdes, en particulier le Δ⁹ THC, ou un protecteur de son métabolisme (revue Piomelli et al., TIPS, 2000), et/ou
- la morphine ou l'un de ses dérivés, et/ou
- un dérivé du Gaba, tel que la gabapentine ou la prégabaline
et un excipient pharmaceutiquement approprié, en particulier un excipient approprié pour une administration par voie orale, nasale, intraveineuse ou transcutanée.

L'invention concerne également l'utilisation d'au moins un dérivé de cannabinoïdes, en particulier le Δ⁹ THC, et/ou la morphine ou l'un de ses dérivés, et/ou un dérivé du Gaba, tel que la gabapentine ou la prégabaline, dans une composition pharmaceutique pour potentialiser l'effet analgésique et/ou antidépresseur des composés de formule (I) tels que définis précédemment.

L'invention concerne aussi l'utilisation d'une combinaison d'au moins un composé de formule (1) tel que défini précédemment et d'au moins un dérivé de cannabinoïdes, en particulier le Δ⁹ THC, et/ou la morphine ou l'un de ses dérivés, et/ou un dérivé du Gaba, tel que la gabapentine ou la prégabaline, pour la préparation d'un médicament destiné au traitement de la dépression et de la douleur, en particulier la douleur aigue, la douleur inflammatoire, la douleur neurogénique, la douleur neuropathique, la douleur psychogène, l'allodynie.

Un autre objet de l'invention est une composition pharmaceutique comprenant
i) au moins un composé de formule (1) tel que défini précédemment
ii) au moins un dérivé de cannabinoïdes, et/ou
iii) la morphine ou l'un de ses dérivés, et/ou
iv) au moins un dérivé du Gaba, tel que la gabapentine ou la prégabaline,
en tant que produits de combinaison pour une utilisation simultanée, séparée ou étalée dans le temps.

La dose efficace d'un composé de l'invention varie en fonction de nombreux paramètres tels que, par exemple, la voie d'administration choisie, le poids, l'âge, le sexe, l'état d'avancement de la pathologie à traiter et la sensibilité de l'individu à traiter. En conséquence, la posologie optimale devra être déterminée, en fonction des paramètres jugés pertinents, par le spécialiste en la matière.

L'invention a également pour objet une méthode de traitement, de l'une quelconque des maladies précédemment citées, comprenant l'administration, chez le patient qui nécessite un tel traitement, d'au moins un des composés selon l'invention ou d'une composition comprenant au moins un de ces composés. Les composés selon l'invention peuvent être utilisés, dans cette méthode, seuls ou en association avec notamment au moins un des composés décrits précédemment.

L'invention sera encore illustrée sans aucunement n'être limitée par les exemples ci-après. La liste des composés préparés est donnée dans le tableau 1. Pour tous les composés décrits dans ces exemples 6, 8, 10, 12, 14, 16 19 et 22.
* R₁ représente le radical -C(O)-O-CH(CH₃)-OC(O)-iPr.
* R₆ représente un hydrogène

**Tableau 1 : radicaux des exemples**

| Exemple | R₂ | R₃ | R₄ | R₅ | R₇ |
|---|---|---|---|---|---|
| 6 | -CH₂-CH₂-S-CH₃ | benzyle | H | H | -CH(CR₃)-OC(O)-O-C₂H₅ |
| 8 | -CH₂-CH₂S-CH₃, | benzyle | H | H | benzyle |
| 10 | -CH₂-CH₂-S-CH₃ | benzyle | H | H | H |
| 12 | -CH₂-CH₂-SO-CH₃ | benzyle | H | H | -CH(CH₃)-OC(O)-O-C₂H₅ |
| 14 | -CH₂-CH₂-SO-CH₃ | benzyle | H | H | - benzyle |
| 16 | -CH₂-CH₂-SO-CH₃ | benzyle | H | H | H |
| 19 | -CH₂-CH₂-S-CH₃ | -(CH₂)₄- | | -CH₂-COOH | H |
| 22 | -CH₂-CH₂-SO-CH₃ | -(CH₂)₄- | | -CH₂-COOH | H |

### Exemple 1: Synthèse de l'ester tert-butyl de l'acide (1 Mercaptométhyl-3-méthylsulfanyl-propyl)-carbamique

Ce composé est préparé en suivant le protocole décrit dans J. Med.Chem., 35, 1992, 2473. Solide blanc ; point de fusion 37°C ; Rf (cyclohexane (CHex)/acétate d'éthyle (AcOEt)) 1/1 =0,73).
HPLC (Kromasil C18, CH₃CN (0,1% TFA) 50 % / H₂O (0,1 % TFA) 50 %) Rt = 15,7 min.
RMN (CDCl₃) δ (ppm) 1,30 (1H, t), 1,52 (9H, s), 1,80-1,90 (2H, m), 2,10 (3H, s), 2,55 (2H, t), 2,80 (2H, t), 3,88 (1H, m), 4,80 (1H, d).

### Exemple 2 : Synthèse de l'acide (2S)-2-Benzyl-3-mercapto-propanoïque

Etape 1 : L'ester méthylique de l'acide 3-Acetylsulfanyl-2-benzyl-propanoïque, obtenu par estérification de l'acide correspondant est traité par l'α-chymotrypsine selon le protocole décrit dans Bioorg. Med. Chem. Lett., 3, (1993), 2681. Rendement 71%; excès énantiomérique ee 88% α_{D}^{20°C}-42,7°.

Etape 2 : Acide (2S)- 2-Benzyl-3-mercapto-propanoïque.
Le composé de l'étape 1 est dissous dans le méthanol dégazé à 0°C. On ajoute sous atmosphère inerte 3 équivalents de NaOH (soude) 1N et le mélange est agité 30 min. à température ambiante. Le mélange est acidifié par HCl 6N et le MeOH est évaporé sous pression réduite. La phase aqueuse est extraite par EtOAc. La phase organique est lavée par une solution saturée de NaCl, séchée sur Na₂SO₄ et évaporée à sec. On obtient une huile jaune. Rendement quantitatif
HPLC (Kromasil C18 (CH₃CN (0,1% TFA) 60% / H₂O (0,1% TFA) 40%) Rt = 4,96 min.
RMN (CDCl₃) δ (ppm) 1,5 (1H, t), 2,7-3,2 (5H, m), 7,25 (5H, m), 12 (1H, s).

### Exemple 3: Synthèse de l'acide 2-(2-tert-Butoxycarbonylamino-4-méthylsulfanyl-butyldisulfanylméthyl)-3-phényl-propanoique

Un mélange de 23 mL de MeOH et 23 mL de THF est refroidi à 0°C sous azote et le chlorocarbonylsulfenylchloride (1,3 mL, 1,1 équivalent) est ajouté. Le mélange est agité pendant 15 min à 0°C pour donner le méthoxycarbonylsulfenylchloride. Le composé de l'exemple 1 (1,06 équivalent) dans 16 mL de THF est ajouté en 1 seule fois. Le mélange est ramené à température ambiante et est agité pendant 30 min. Cette solution est ajoutée goutte à goutte à une solution du composé de l'exemple 2 (1 équivalent) dans 100 mL de CHCl₃ dégazé en présence de Et₃N (1 équivalent). Le mélange est agité 1h à température ambiante puis le solvant est évaporé à sec. Le résidu est repris dans le CH₂Cl₂ et la phase organique est lavée par une solution à 10% d'acide citrique, une solution saturée de NaCl, séchée sur Na₂SO₄. Après filtration et évaporation à sec on obtient une huile jaune pale qui est utilisée telle quelle pour la suite des réactions. Rendement 98%
HPLC (Kromasil C18 (CH₃CN (0,1% TFA) 70% / H₂O (0,1% TFA) 30%) Rt=7,71 min.
RMN (DMSOd6) δ (ppm): 1,35 (9H, s), 1,7 (2H, m), 2,0 (3H, s), 2,4 (2H, t), 2,7- 3,0 (5H, m), 3,70 (1H, s), 6,80 (1H, d), 7,20 (5H, m).

### Exemple 4: Synthèse du trifluoroacétate de l'ester 1-éthoxycarbonyloxy-éthyl de l'acide Amino-ascétique

La Boc-Gly (4,88g) et la Et₃N (triéthylamine) (4,65 mL, 1,2 équivalent) sous dissous dans 25 mL d'acétate d'éthyle. L'éthyl-1-chloroéthylcarbonate (préparé selon Barcelo et al., Synthesis, 1986, 627) (4,68 g, 1,1 équivalent) et Nal (1,64 g, 0,4 équivalent) sont ajoutés et le mélange est porté 16 h au reflux. Le précipité est filtré, et 15 mL d'acétate d'éthyle et 20 mL d'eau sont ajoutés au filtrat. La phase organique est séparée et la phase aqueuse est extraite 3 fois à l'acétate d'éthyle. Les phases organiques réunies sont lavées par une solution d'acide citrique à 10%, une solution de NaHCO₃ à 10%, une solution de NaCl saturée, séchée sur Na₂SO₄, filtrées et évaporées à sec. On obtient une huile orange, 7,8 g Rendement 95%. Rf (cHexane/ AcOEt : 8/2) 0,40.
Le Produit huileux de l'étape 1 est mis en solution dans 24 mL de CH₂Cl₂ et 21,3 mL de TFA. Après agitation 1h à température ambiante, le mélange réactionnel est évaporé à sec. L'huile jaune obtenue est reprise dans un mélange éther/hexane; Le précipité formé est lavé 3 fois par le mélange éther/hexane, puis est séché. Solide blanc 7,2 g (Rendement 85%)
RMN (DMSOd6) δ (ppm): 1,2 (3H, q), 1,5 (3H, d), 3,90 (2H, dd), 4,10 (2H, q), 6,75 (1H, q), 8,37 (3H, s).

### Exemple 5 : ester 1-éthoxycarbonyloxy-éthyl de l'acide [2-(2-tert-Butoxycarbonylamino-4-méthylsulfanyl-butyldisulfanylméthyl)-3-phényl-propionylamino]-acétique

Le composé de l'exemple 3 (2g), le composé de l'exemple 4 (1,47 g, 1,1 équivalent), le TBTU (1,62 g, 2 équivalents)- et la DIEA (2,57 ml) sont solubilisés dans 20 mL de DMF. Le mélange est agité 15 min à température ambiante, puis le DMF est évaporé sous pression réduite. Le résidu est repris dans l'acétate d'éthyle et la phase organique est lavée par une solution d'acide citrique à 10%, une solution de NaHCO₃ à 10% et une solution saturée de NaCl. La solution est séchée sur Na₂SO₄, filtrée et évaporée à sec. Le produit brut est purifié sur colonne de silice (cHexane/AcOEt : 6/4). Solide blanc 2,06 g (Rendement 75%)
HPLC Kromasil C18 (CH₃CN (0,1% TFA) 70% / H₂O (0,1% TFA) 30 %) Rt=11,2 min. Masse (M+H)⁺=631.

### Exemple 6: ester 1-(1-{2-[(1-éthoxycarbonyloxy-éthoxycarbonylméthyl)-carbamoyl]-3-phényl-propyldisulfanylméthyl}-3-méthylsulfanyl-propylcarbamoyloxy)-éthyl de l'acide isobutrique.

Le composé de l'exemple 5 (1,7 g) est solubilisé dans 17 mL d'acide formique, et le mélange est agité 2h à température ambiante. L'acide formique est évaporé sous vide, le résidu est repris 3 fois par du cyclohexane et évaporé à sec. Huile jaune 1,5g (Rendement 97%).

Le formiate obtenu est solubilisé dans 20 mL de CH₂Cl₂ et 2,4 mL de DIEA_(5 équivalents). On ajoute 1,15 g (1,5 équivalent) de l'ester 1-(2,5-dioxo-cyclopentyloxycarbonyloxyl-éthy de l'acide isobutyrique et le mélange est agité 1h à température ambiante. Le solvant est évaporé et le résidu est repris par l'acétate d'éthyle. La phase organique est lavée par de l'eau, par une solution d'acide citrique à 10%, une solution saturée NaCl, séchée sur Na₂SO₄ puis filtrée et évaporée à sec.
Purification par HPLC sur colonne semipreparative Kromasil C18 (CH₃CN (0,1% TFA) / H₂O (0,1% TFA) : 70/30). Solide blanc 0,96 g (Rendement 50%)
HPLC (Kromasil C18 (CH₃CN (0,1% TFA) / H₂O (0,1 %TFA) : 70/30) Rt=10,98 min.
Masse (M+H)⁺=577
RMN (CDCl₃) δ (ppm): 1,10 (6H, d), 1,25 (3H, t), 1,5 (2x3H, d), 1,65-1,85 (2H, m) 2,47 (4H, m), 2,5 (1H, m), 2,5-3,00 (5H, m),3,90-4,00 (3H, m), 4,15 (2H, q), 4,9 (1H, d), 6,4 (1H, t), 6,75 (2x 1H, q), 7,20 (10H, m).

### Exemple 7: Ester benzylique de l'acide [2-(2-tert-Butoxycarbonylamino-4-méthylsulfanyl-butyldisulfanylméthyl)-3-phényl-propionylamino]-acétique

Le composé de l'exemple 3 (4g) et l'ester benzylique de la glycine sous forme de sel d'APTS (4,55 g, 1,5 équivalents) sont mis en solution dans le DMF (20 mL). On ajoute le TBTU (3,43 g, 1,2 équivalent) et la DIEA (5 mL). Le mélange est agité 15 min à température ambiante. Puis la réaction est traitée selon le protocole décrit dans l'exemple 5. On obtient un solide blanc 5,3g (Rendement 99%) Masse (M+H)⁺=593. HPLC (Kromasil C18, CH₃CN (0,1% TFA) / H₂O (0,1% TFA) : 70/30) Rt=12,9 min RMN (CDCl₃) δ (ppm) 1,4 (9H, s), 1,7-1,9 (2H, m), 2,10 (3H, s), 2,5 (2H, m), 2,8- 3,10 (7H, m), 3,80-4,10 (3H, m), 4,70 (1H, d), 5,15 (2H, s), 6,60 (1H, t), 7,20-7,40 (10H, m).

### Exemple 8 : Ester 1-{1-[2-(benzyloxycarbonylméthyl-carbamoyl)-3-phényl-propyldisulfanylméthyl]-3-méthylsulfanyl-propylcarbamoyloxy} -éthyl de l'acide isobutyrique

Le composé de l'exemple 7 (724 mg) est solubilisé dans 5 mL de TFA et 5 mL de CH₂Cl₂. Le mélange est agité 3 h à 0°C. Le mélange réactionnel est évaporé sous pression réduite, et le résidu est repris par l'eau et lyophilisé.
Solide blanc 720 mg (Rendement 97%) Masse (M+H)⁺ 607.
HPLC (Kromasil C18, (CH₃CN (0,1% TFA)/H₂O (0,1 %TFA) : 50/50) Rt=6,2 min
Le trifluoracétate obtenu (720 Mg) est solubilisé dans 10 mL de CH₂Cl₂. On ajoute de la DIEA (1 mL, 5 équivalents) puis 500 mg (1,5 équivalenst) de ester 1-(2,5-dioxo-cyclopentyloxycarbonyloxy)-éthyl de l'acide isobutyrique et le mélange est agité 1h à température ambiante. Le solvant est évaporé et le résidu es repris par l'acétate d'éthyle. La phase organique est lavée à l'eau, par une solution saturée de NaCl, et séchée sur Na₂SO₄. Après filtration et évaporation à sec on obtient un composé huileux qui est purifié par HPLC sur colonne semi préparative Kromasil C18, CH₃CN (0,1% TFA/ H₂O (0,1% TFA) 70/30. Solide blanc 390 mg (Rendement 48,5%). Masse (M+H)⁺=651. HPLC Kromasil C18 (CH₃CN (0,1% TFA) 70 %/H₂O (0,1% TFA) 30%) Rt=12,5 min. RMN (CDCl₃) δ (ppm) : 1,1 (6H, d), 1,5 (3H, d), 1,7-1,9 (2H, m), 2, (3H, s), 2,5 (3H, m), 2,7-3,0 (7H, m), 3,7-4,2 (3H, m), 4,95 (1H, d), 5,15 (2H, s), 6,4 (1H, t), 6,7 (1H, q), 7,2 (10H, m).

### Exemple 9 : ester tert-butyl de l'acide [2-(2-tert-Butoxycarbonylamino-4-méthylsulfanyl-butyldisulfanylméthyl)-3-phényl-propionylamino]-acétique

Le composé de l'exemple 3 (1g) et l'ester tert-butylique de la glycine (563 mg, 1,5 équivalents) sont mis en solution dans 5 mL de -DMF en présence de TBTU (857 mg, 1,2 équivalents) et DIEA (1,24 mL). Le mélange est agité 15 min à température ambiante, puis le mélange réactionnel est traité comme décrit dans l'exemple 5. On obtient un solide blanc 918 mg (Rendement 74%).
HPLC Kromasil C18 (CH₃CN (0,1% TFA) 70%/H₂O (0,1% TFA) 30%) Rt=13,3 min.
Masse (M+H)⁺=559

### Exemple 10 : Ester 1- {1-[2-(carboxymétyl-carbamoyl)-3-phényl-propyl-disulfanylméthyl]-3-méthylsulfanyl-propylcarbamoyloxy}-éthyl de l'acide isobutyrique

Le composé de l'exemple 9 (914 mg) est solubilisé dans 5 mL de CH₂Cl₂ et 5mL de TFA et le mélange est agité 3h à température ambiante. Après évaporation à sec le résidu est repris par de l'eau et lyophilisé. Solide blanc 844 mg (Rendement quantitatif). Le trifluoroacétate (844mg) est solubilisé dans 10mL de CH₂Cl₂. On ajoute 1,34 mL (5 équivalents) de DIEA et 670 mg (1,5 équivalents) de l'ester 1-(2,5-dioxo-cyclopentyloxylcarbonyloxy)-éthyl de l'acide isobutyrique et le mélange est agité 1h à température ambiante. Le mélange réactionnel est ensuite traité comme décrit dans l'exemple 6. Le produit brut est purifié par HPLC sur colonne Kromasil C18 semi préparative (CH₃CN (0,1% TFA/H₂O (0,1% TFA): 55/45. Solide blanc 400 mg (Rendement 44%).

HPLC Kromasil C18 (CH₃CN (0,1% TFA) 60%/H₂O (0,1% TFA) 40%) Rt= 7,80 min Masse (M+H)⁺ =561.
RMN (CDCl₃) δ (ppm) : 1,0 (6H, dd), 1,4 (3H, dd), 1,6-1,75 (2H, m), 2,0 (3H, s), 2,5 (3H, m), 2,8-3,1 (7H, m), 3,8-4,1 (3H, m), 5,0 (1H, d), 6,7 (1H, q), 7,2 (5H, m).
Le sel de sodium du composé 10 est obtenu en solubilisant l'acide dans l'acétonitrile puis en ajoutant 1 équivalent de NaHCO₃ en solution dans l'eau. La solution ainsi obtenue est lyophilisée. Solide blanc (Rendement 96%)

### Exemple 11 : Ester 1-éthoxycarbonyloxy-éthyl de l'acide [2-(2-tert-butoxycarbonyl-amino-4-méthanesulfinyl-butyldisulfanylméthyl)-3-phényl-propionylamino]-acétique

Le composé de l'exemple 5 (2g) est solubilisé dans 40 mL d'éthanol. On ajoute 32 mL d'une solution de NaIO₄ 0,2M (2 équivalents) à 0°C et le mélange est agité pendant 3h à 0°C. Le précipité est filtré et le filtrat est évaporé à sec. Le résidu est repris dans l'acétate d'éthyle et la phase organique est lavée à l'eau, par une solution saturée de NaCl séchée sur Na₂SO₄. Après filtration et évaporation, le produit brut est purifié par chromatographie.
Solide blanc 1,5 g (Rendement 70%)
HPLC (Kromasil C18 (CH₃CN (0,1%.TFA) 60%/H₂O (0,1% TFA) 40%) Rt=8,3 min.
Masse (M+H)⁺= 635

### Exemple 12 : Ester 1-(1-{2-[(1-éthoxycarbonyloxy-éthoxycarbonylméthyl)-carbamoyl]-3-phényl-propyldisulfanylméthyl}-3-méthanesulfinyl-propylcarbamoloxy)-éthyl de l'acide isobutyrique

Le composé de l'exemple 11 (1,5 g) est solubilisé dans 20 mL d'acide formique et le mélange est agité 1h à température ambiante. L'acide formique est évaporé sous vide et le résidu est repris par de l'eau et lyophilisé.
Solide blanc 1,38 g.
Le formiate obtenu (1,38 g) est solubilisé dans le CH₂Cl₂ et on ajoute le carbamate (1,5 équivaments) (ester 1-(2,5-dioxo-cyclopentyloxycarbonyloxy)-éthyl de l'acide isobutyrique) et la DIEA (3 équivalents). Le mélange est agité 1h à température ambiante puis le solvant est évaporé sous pression réduite. Le résidu est repris par de l'acétate d'éthyle. La phase organique est lavée avec une solution d'acide citrique à 10%, une solution saturée de NaCl, séchée sur Na₂SO₄, filtrée et évaporée à sec. Le produit brut est purifié par HPLC sur colonne Kromasil semi préparative (CH₃CN (0,1% TFA)/H₂O (0, 1 % TFA) :50/50
Rendement 54%.
HPLC Kromasil C18 (CH₃CN (0,1% TFA) 50%/H₂O (0,1% TFA) 50%) Rt=14,55 min. Masse (M+H)⁺ = 693.
RMN (DMSOd6) δ (ppm): 1,0 (6H, dd), 1,15 (3H, t), 1,4 (2x3H, d), 1,65-1,90 (2H, m), 2,4-3,0 (13H, m), 3,75 (1H), 3,9 (2H, d), 4,0 (2H, q), 5,1 (2H, s), 6,65 (2H, m), 7,1-7,3 (5H, m), 7,5 (1H, d), 8,5 (1H, t).

### Exemple 13: Ester benzylique de l'acide [2-(2-tert-butoxycarbonylamino-4-méthanesulfinyl-butyldisulfanylméthyl)-3-phényl-propionylamino]-acétique Le composé de l'exemple 7 (5,3 g) est traité dans les conditions de l'exemple 1 pour conduire à 5,19 g du composé attendu. Rendement 95%

HPLC Kromasil C18 (CH₃CN (0,1% TFA) 60%/H₂O (0,1% TFA) 40%) Rt=7,3 min

### Exemple 14: Ester 1-{1-(2-benzyloxycarbonylméthyl-carbamoyl)-3-phényl-propyldisulfanylméthyl]-3-méthanesulfinyl-propylcarbamoyloxy}-éthyl de l'acide isobutyrique

Le composé de l'exemple 13 (3,1g) est traité par 50mL d'acide formique et la réaction est traitée comme décrit dans l'exemple 12. Solide blanc 2,85 g (Rendement 99%)

Le formiate obtenu (1,41 g) est solubilisé dans un mélange 20 mL de dioxanne et 20 mL d'eau. On ajoute 1,1 g (8 équivalents) de NaHCO₃ et 1,13g (1,5 équivalents) d'ester 1-(4-nitro-phénoxycarbonyloxy)-éthyl de l'acide isobutyrique et le mélange est agité pendant 72 h à température ambiante. Le dioxanne est évaporé à sec et la phase aqueuse est extraite par l'acétate d'éthyle. La phase organique est lavée par une solution saturée de NaCl, séchée sur Na₂SO₄. Après filtration et évaporation à sec le produit est purifié par HPLC semi préparative sur colonne Kromasil C18 (CH₃CN (0,1% TFA)/H₂O (0,1% TFA) :55/45. Solide blanc 1,0 g (Rendement 59%)
HPLC Kromasil C18 (CH₃CN (0.,% TFA) 60%/H₂O (0,1% TFA) 40%) Rt=8,21 min. Masse (M+H)⁺=667
RMN (DMSOd6) δ (ppm): 1,0 (6H, dd), 1,4 (3H, d), 1,65-1,90 (2H, m), 2,4-3,0 (13H, m), 3,75 (1H), 3,9 (2H, d), 5,1 (2H, s), 6,65 (1H, m), 7,1-7,3 (10H, m), 7,5 (1H, d), 8,5 (1H, t).

### Exemple 15: Ester tert-butyl de l'acide [2-(2-tert-butoxycarbonylamino-4-méthanesulfinylméthyl)-3-phényl-propionylamino]-acétique

Le composé de l'exemple 9 (745 mg) est traité dans les conditions de l'exemple 11 pour conduire à 781 mg (rendement quantitatif) du produit attendu.
HPLC Kromasil C18 (CH₃CN (0, 1 % TFA) 70%/H₂O (0, 1 %TFA) 30%) Rt = 4,65 min. RMN (DMSOd6) δ (ppm) : 1,4 (18H, s), 1,7-1,9 (2H, m), 2,5-3 (12H, m), 3,7 (2H, d + 1 H, m), 6,9 (1H, d), 7,2 (5H, m), 8,4 (1H, t).

### Exemple 16: Ester 1-{1-2[2-(carboxyméthyl-carbamoyl)-3-phényl-propyldisulfanyl-méthyl]-3-méthanesulfinyl-propylcarbamoyloxy}-éthyl de l'acide isobutyrique

Le composé de l'exemple 15 (760 mg) est traité par 5 mL de TFA dans 5 mL de CH₂Cl₂ et la réaction est agitée 3h à température ambiante. Les solvants sont ensuite évaporés sous pression réduite, le résidu est repris dans l'eau et lyophilisé. On obtient un produit blanc (686 mg; Rendement 99%).
Le composé obtenu (686 mg) est solubilisé dans 10 mL d'eau et 10 mL de dioxanne. On ajoute 572 mg (8 équivalents) de NaHCO₃ et 587 mg (1,5 équivalents) d'ester (1-(4-nitro-phénoxycarbonyloxy)-éthyl de l'acide isobutyrique. Le mélange est agité 12h à température ambiante. Le dioxanne est évaporé sous pression réduite et la phase aqueuse est extraite par l'acétate d'éthyle. La phase organique est lavée par une solution de NaCl saturée, séchée sur Na₂SO₄, filtrée et évaporée à sec. Le produit brut est purifié par HPLC semi préparative sur colonne Kromasil C18 (CH₃CN (0,1% TFA)/ H₂O (0,1% TFA): 35/65. Solide blanc 212 mg. Rendement 28,1%)
HPLC Kromasil C18 (CH₃CN (0,1% TFA) 40%/H₂O (0,1% TFA) 60%) Rt =11,98 min. Masse (M +H)⁺= 577
RMN (DMSOd6) δ (ppm) : 1,0 (2x3H, d), 1,4 (3H, d), 1,7-1,9 (2H, m), 2,5-3,0 (13H, m), 3,7 (2H, d =1H, m), 6,65 (1H, m), 7,2 (5H, m), 7,5 (1H, d), 8,4 (1H, t).

### Exemple 17 : Ester tert-butyl de l'acide cyclopentane-1,1-dicarboxylique

L'ester t-butylique de l'acide cyclopentane carboxylique (préparé selon J. Med. Chem., 1994,37,2461-2476) (10g) est solubilisé dans 50 mL de THF sous azote. On ajoute à -30°C la solution de LDA (1,3 équivalent) préparée à partir de 10,71 mL de diisopropylamine et de 47,75 mL de Butyllithium 1,6 M dans l'hexane. Après agitation 30 min à -30°C on fait barboter du CO₂ pendant 15min à même température. Le mélange est ramené à -5°C et 100mL d'eau sont ajoutés. On évapore le THF, la phase aqueuse est extraite 2 fois à l'acétate d'éthyle puis est acidifiée à pH 1. Après extraction 3 fois à l'acétate d'éthyle, la phase organique est lavée par une solution saturée de NaCl, séchée sur Na₂SO₄, filtrée et évaporée à sec. Solide blanc 10g (Rendement 80%).

### Exemple 18 : Ester tert-butyl de l'acide 1-Mercaptomethyl-cyclopentanecarboxylique

Le composé précédent (7,9g) est solubilisé dans 70 mL de THF. A -10°C, on ajoute 5,13 mL (1 équivalent) de triéthylamine et 4,78 mL (1 équivalent) d'isobutylchloroformate. Après 2 min de réaction, le précipité formé est filtré et on ajoute à la solution organique 4,88g de NaBH₄ (3,5 équivalents) puis 22 mL de MeOH goutte à goutte. La solution est ramenée à 0°C et acidifiée par HCl 1N. Après extraction au CH₂Cl₂, lavage et séchage, la phase organique est évaporée à sec. Huile jaune pale (6,52 g, Rendement 88%)

L'alcool obtenu (5 g) est solubilisé dans l'éther (65 mL). On ajoute 2,12 mL (1,1 équivalent) de chlorure de mésyle et 4 mL de triéthylamine. Après agitation 3h à température ambiante, la phase éthérée est lavée, séchée et évaporée à sec. Huile jaune pale (6,4g, Rendement 92%)

Le mésylate (6,4g) est solubilisé dans le diméthylformamide (100mL) On ajoute 3,18 g de K₂CO₃ et 3,61 mL d'acide thioacétique et le mélange est agité 5h à 100°C. Le DMF est évaporé à sec et le résidu repris par l'acétate d'éthyle et HCl 1N. La phase organique est lavée, séchée et évaporée à sec.

L'huile brune obtenue est traitée par un mélange 50/50 de CH₂Cl₂/ TFA à température ambiante. Le mélange est évaporé à sec, repris 3 fois par du cyclohexane et évaporé à sec. Produit solide rouille (rendement quantitatif). L'acétylthioacide formé est solubilisé dans 50 mL de MeOH et 50 mL de NaOH 1N est ajouté. Le mélange est agité 3 h à température ambiante puis est acidifié par HCl 1N. Le méthanol est évaporé, le résidu repris par EtOAc et la phase organique est lavée et séchée : Huile jaune pale, rendement quantitatif.
RMN (CHCl₃) δ (ppm) : 1,6-1,8 (6H m+SH), 2,15 (2H m), 3,2 (2H,s).

### Exemple 19: Acide 1-(2-tert-Butoxycarbonylamino-4-methylsulfanyl-butyldisulfanyl-methyl)-cyclopentanecarboxylique

En suivant le protocole de l'exemple 3 et en remplaçant le composé de l'exemple 1 par le composé de l'exemple 18, on obtient le produit attendu. Solide blanc, 3,8g (rendement 75%). HPLC (Kromasil C18, CH₃CN(0,1%TFA)/H₂O(0,1% TFA) :50/50) Rt 31,6 min. Masse (M+H)⁺=410.

### Exemple 20 : Ester di-tert-butyl de l'acide 2-{[1-(2-tert-Butoxycarbonylamino-4-methylsulfanyl-butyldisulfanylmethyl)-cyclopentanecarbonyl]-amino}-succinique

En suivant le protocole de l'exemple 5 et en remplaçant le composé de l'exemple 4 par le diester t-butylique de l'acide aspartique, on obtient le composé attendu avec un rendement de 80 %. Solide blanc. Masse (M+H)⁺ = 637,3
HPLC (Kromasil C18, CH₃CN(0,1%TFA)/H₂O(0,1% TFA) :80/20) Rt 31,6 min. RMN (CDCl₂) δ (ppm) :1,4 (27 H), 1,7-1,9 (8H m), 2,1 (5H m), 2,5 (2H m) 2,8-3,1 (6H m), 4,1 (1H m), 4,6 (1H m), 4,7 (1H d), 6,6 (1H d).

### Exemple 21: Ester di-tert-butyl de l'acide 2-{[1-(2-tert-Butoxycarbonylamino-4-methanesulfinyl-butyldisulfanylmethyl)-cyclopentanecarbonyl]-amino}-succinique

En suivant le protocole décrit dans l'exemple 11, le composé de l'exemple 20 conduit au produit attendu avec un rendement de 81%.
Masse (M+H)⁺ = 653,3

### Exemple 22 : Acide 2-({1-[2-(1-Isobutyryloxy-ethoxycarbonylamino)-4-methanesulfinyl-butyldisulfanylmethyl]-cyclopentanecarbonyl}-amino)-succinique

En suivant le protocole de l'exemple 12, le composé de l'exemple 21 conduit au produit final attendu avec un rendement de 63%.
HPLC (Kromasil C18 CH₃CN(0,1% TFA)/H₂O(0,1% TFA) : 70/30) Rt 4,3 min
Masse (M+H)⁺ = 599,2
RMN (DMSOd6) δ (ppm) : 1,0 (6H d), 1,4 (3H d), 1,7-1,9 (8H m), 2,1 (2H m), 2,5-3,0 (12H m), 3,7 (1H m), 4,6 (1H m), 6,65 (1H m), 7,5 (1H d), 8,4 (1H d).

### Exemple 23: Résultats pharmacologiques

Les molécules de la présente invention ont été étudiées pour leur action analgésique sur les modèles animaux les plus prédictifs de la réponse chez l'homme. Les tests privilégiés sont ceux qui s'adressent aux douleurs neuroinflammatoires (NI) et neuropathiques (NP) chez le rat et la souris.
Les molécules de la présente invention se sont révélées actives sur les tests suivants. Chez le rat : i) douleur neuropathique évoquée par injection intraplantaire de carragénine ou de l'adjuvant de Freund (Desmeules A. et al. Pain (1993), 53, 277-285) ; ii) neuropathie (NP) diabétique induite par la préadministration de streptozocine (Condore-Civiale et al., Br. J. Pharmacol. (2001), 67, 1301-1308) ; iii) neuropathie déclenchée par pré-administration d'un anticancéreux, la vincritine (Authier et al., Neurotoxicology (2003), 4, 797-805).
Chez la souris : i) hyperalgésie et allodynie neurophatique et neuroinflammatoire induite par pré-administration de cellules tumorales dans la moelle tibiale, modèle de l'ostéosarcome (Menendez L. et al., Brain Res. (2003), 969, 102-109) ; ii) douleur provoquée par l'administration de formaline dans la patte et étude de la réponse analgésique dans la première phase (NT) ; iii) hyperalgésie et allodynie produites par compression partielle et unilatérale du nerf sciatique (modèle de Seltzer) (Bennett G.J. and Xie Y.K., Pain (1998) 33, 87-107).
Les techniques utilisées dans ces tests sont décrites en détail et répertoriées dans des revues telles que : M.J. Millan. The induction of pain: an integrative review, in Progress in Neurobiology (1999), 52, 1-164.
A titre d'exemples on trouvera ci-dessous plusieurs études.

### A/ Test à la formaline (phase 1)

Les molécules ont été étudiées à deux temps, 90 et 150 minutes, de manière à observer leur durée d'action.

### Description du test.

Les animaux (souris mâle OF1) proviennent de l'élevage Charles River (France) et pèsent 25-35 g au début de l'expérience. Le poids de chaque souris est pris en compte pour l'administration du produit.

Le test est basé sur le protocle décrit par S. HUNSKAAR et al., Formalin test in mice, a usefull technique for evaluating mild analgesics, J. Neurosci. Methods (1995), 14, 69-75.

Les souris (n=8) sont placées individuellement dans une enceinte transparente (50x25 cm) et habituées à cet environnement pendant 20 minutes. Après cette période, 20 µl de formaline (5% HCHO) en solution dans du sérum physiologique (H₂O, NaCl 0,9%), sont injectés par voie sous-cutanée sur la face plantaire de la patte droite de l'animal. On utilise une seringue de 26 connectée à une micro-seringue. Chaque souris est ensuite immédiatement replacée dans l'enceinte du test et des réponses douloureuses (nociceptives) sont mesurées durant 5 minutes (phase précoce). Seuls les lèchements de la patte sont comptés.

L'activité analgésique est testée après gavage des animaux à différents temps (généralement 20 mn, 90 mn et 150 mn) avant l'injection de formaline, par :
- le véhicule seul (éthanol, méthylcellulose 0,5% dans l'eau)
- le véhicule et un composé de l'invention (50 mg/kg)

L'action analgésique du produit est mesurée par la diminution du nombre de lèchements de la patte lésée, par rapport au nombre de lèchements de l'animal qui a reçu le véhicule seul.

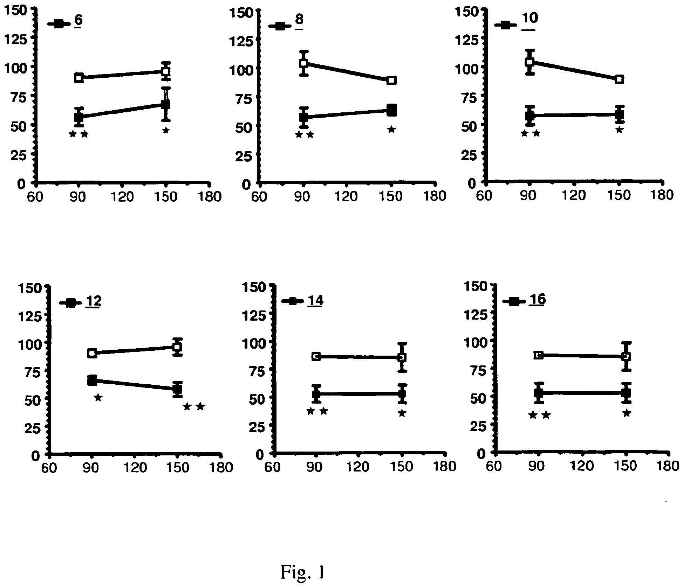

Les résultats sont figurés pour les six composés des exemples 6, 8, 10, 12, 14 et 16 sur la **figure 1****.**

Les six composés montrent des effets analgésiques puissants (40 à 60%) caractérisés par un abaissement très significatif du nombre de lèchements par rapport au véhicule (contrôle) et les effets sont à peu près constants durant la période du test. L'action analgésique est bloquée par une pré-administration d'un antagoniste, le méthylnaloxonium qui, à la dose utilisée (2 mg/kg), est incapable de franchir la barrière hématoméningée (Milne R.J. et al., Neurosci. Lett. (1990), 114, 25-32), démontrant que l'activité de ces molécules s'exerce au niveau périphérique (nocicepteurs) où ils augmentent les enképhalines libérées au site lésé.

### B/ Etude comparative de l'effet analgésique du composé de l'exemple 6 et de la molécule de référence (Composé 15 de la demande internationale W02007/048787

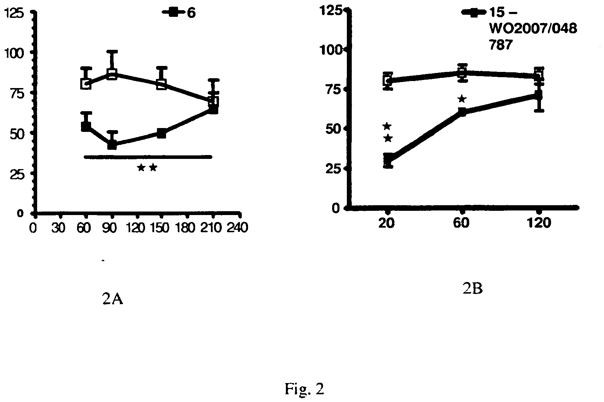

La présente invention se caractérise par la mise au point de molécules possédant des propriétés analgésiques au moins égales à celles des composés décrits dans la demande internationale W02007/048787, mais une durée d'action considérablement allongée. Ceci est bien mis en évidence dans le test à la formaline (dont le protocole a été décrit précédemment) puisque la molécule de référence :
Composé 15 = NH₂-CH(CH₂CH₂SCH₃)-CH₂-S-S-CH₂-CH(CH₂C₆H₅)-CONH-CH₂-CONH-CO-CH(CH₃)-O-CO-OCH₂CH₃ (W02007/048787) ne possède plus aucune activité à 120 minutes alors que le composé de l'exemple 6 atteint au contraire son maximum d'activité analgésique entre 90 et 150 minutes **(****figure 2****)**.

### C/ Effets antiallodynique et antihyperalgésique du composé de l'exemple 10 après administration orale chez la souris.

Ce test a été décrit en détail par A.B. Malmberg et A.I. Basbaum, Partial Sciatic nerve injury in the mouse as a model of neuropathic pain : behavioural and neuroanatomical correlates. Pain, (1998) 76, 215-222.

Il a été réalisé sur des souris mâles OF1 (Charles River), 18-20 g, n=39, par ligature partielle du nerf sciatique du côté ipsilatéral. Les animaux sont testés dans la période (3-26 jours) après l'opération.

La mesure de l'hyperalgésie a été effectuée selon la méthode décrite par K. Hargreaves et al., A new sensitive method for measuring thermal nociception in cutaneous hyperalgesia, Pain, (1988), 32, 77-88, en utilisant comme source de chaleur, l'appareil « Plantar test » (Bioseb, France). L'intensité du stimulus nociceptif est calibrée à 8-10 sec avec un seuil d'arrêt automatique (cut-off time) à 20 sec. La moyenne des retraits de la patte induite par la chaleur, a été mesurée sur les pattes ipsilatérales (nerf endommagé) et contralatérales (nerf intact). Chaque mesure est effectuée 3 fois sur chaque patte. L'allodynie mécanique est mesurée comme décrit par S.R. Chaplan et al., Quantitative assessement of tactile allodynia in the rat paw, J. Neurosci. Meth. (1994), 53, 55-63. Les pattes ipsitatérales (lésion) et contralatérale (contrôle) sont testées comme précédemment. L'effet antiallodynique mécanique est mesuré par la méthode de Von Frey avec des filaments de taille croissante exercant une pression également croissante.

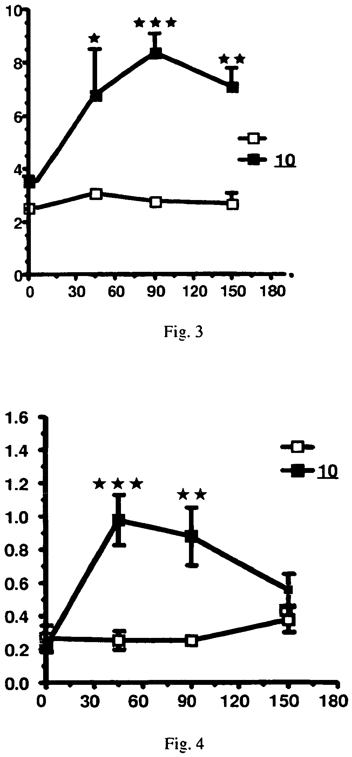

### Effet antihyperalgésique : figure 3

Les résultats de la figure 3 montrent que administré p.o, le composé de l'exemple 10 produit une diminution significative très importante (65-100%) de l'hyperalgésie thermique induite par la ligature partielle du nerf sciatique dans la période 45-150 mn avec un effet maximum de 100% à 80 mn (8,2 ± 0,9 s vs 8,3 s). Il est probable que l'effet doit être encore significatif à 180 mn.

### Effet Anti-allodynique : figure 4

L'effet du composé de l'exemple 10 sur l'allodynie mécanique est mesuré par le test de Von Frey. Les résultats montrent un effet anti-allodynique significatif de longue durée avec un maximum à 60 mn correspondant à 75% de la réponse maximale (contrôle non traité).

### Légende des figures:

Dans toutes les figures, les analyses statistiques (p, test de Student) sont indiquées de la manière suivante :
★ p < 0,1 versus contrôle
★★ p < 0,01 versus contrôle
★★★ p < 0,001 versus contrôle

**Figure 1** : nombre de lèchements (lèchement, sec) de la patte en fonction du temps (minutes) après administration par voie orale du véhicule (□) ou d'un composé selon l'invention (■).
Abscisse : temps en minutes ; ordonnée : nombre de lèchement sec
1A : composé de l'exemple 6 ; 1B : composé de l'exemple 8 ; 1C : composé de l'exemple 10 ; 1D : composé de l'exemple 12 ; 1E : composé de l'exemple 14 ; 1F : composé de l'exemple 16.

**Figure 2** **:** nombre de lèchements (lèchement, sec) de la patte en fonction du temps (minutes) après administration par voie orale du véhicule (□) ou d'un composé selon l'invention ou de référence (■).
Abscisse : temps en minutes ; ordonnée : nombre de lèchement sec
2A : composé de l'exemple 6 ; 2B : composé 15 de la demande WO2007/048787

**Figure 3** **:** Modèle de douleur neuropathique : ligature partielle du nerf sciatique (souris). Réponse générée par l'administration per os du véhicule (□) ou du composé de l'exemple 10 (■) : retrait de la patte (en secondes) en fonction du temps (en minutes).
Composé 10 : 50 mg/Kg
Véhicule : EtOH/methylcellulose 0,5% (1,5/98,5)
Tests effectués à J14 post chirurgie ; patte ipsilatérale
Abscisse : temps en minutes, ordonnée : retrait de la patte en secondes
Plantar test : évaluation de l'hyperalgie thermique
Moyenne des pattes contralatérales : composé 10 à 90 mn = 8,3 s.

**Figure 4** **:** von Frey test : pression de von Frey (g) en fonction du temps (min) après administration par voie orale du véhicule (□) ou du composé de l'exemple 10 (■).
Composé 10 : 50 mg/Kg
Véhicule : EtOH/methylcellulose 0,5% (1,5/98,5)
Abscisse : temps en minutes, ordonnée : pression de von Frey en grammes.
Tests effectués à J 14 post chirurgie.

## Revendications

1. Composé répondant à la formule (1) suivante :
R₁NH-CH(R₂)-CH₂-S-S-CH₂-C(R₃)(R₄)-CONH-C(R₅)(R₆)-COOR₇
dans laquelle :
R₁ représente un groupement (acyloxy)alkyl carbamate -C(O)-O-C(R₈)(R₉)-OC(O)-R₁₀, dans lequel
- R₈ et R₉ représentent indépendamment l'un de l'autre un hydrogène, un groupement alkyle, aryle, arylalkyle, cycloalkyle, cyclohétéroalkyle, hétéroalkyle, hétéroaryle ou hétéroaryalkyle ; ou
- Pris ensemble, R₈ et R₉ peuvent former un cycloalkyle à 5 ou 6 chaînons ;
- R₁₀ représente un groupement alkyle, aryle, arylalkyle, cycloalkyle, cyclohétéroalkyle, hétéroalkyle, hétéroaryle ou hétéroaryalkyle ;
R₂ représente :
- une chaîne hydrocarbonée saturée, linéaire ou ramifiée, comportant de 1 à 6 atomes de carbone, éventuellement substituée par :
* un radical OH, OR₁₁, SH, SR₁₁ ou S(O)R₁₁, dans chacun de ces radicaux R₁₁ représente une chaîne hydrocarbonée linéaire ou ramifiée de 1 à 4 atomes de carbone, un radical phényle ou un radical benzyle,
* un radical phényle ou benzyle, éventuellement substitué par :
• 1 à 5 atomes d'halogène, notamment le fluor,
• un radical OH, OR₁₁, SH, SR₁₁, S(O)R₁₁, R₁₁ ayant la même signification que précédemment,
- un radical méthylène substitué par un hétérocycle à 5 ou 6 chaînons, aromatique ou saturé, possédant comme hétéroatome, un atome d'azote ou de soufre, éventuellement oxydé sous forme N-oxyde ou S-oxyde,
lorsque R₄ représente un atome d'hydrogène, R₃ représente :
- un radical phényle ou benzyle éventuellement substitué par :
* 1 à 5 atomes d'halogène ;
* un radical SR₁₁, S(O)R₁₁, ou OR₁₁, R₁₁ ayant la même signification que précédemment ;
* un groupement amino éventuellement mono- ou disubstitué par un groupement aliphatique, cyclique ou linéaire, de 1 à 6 atomes de carbone ;
- un hétéroaryle à 5 ou 6 chaînons, l'hétéroatome étant un oxygène, un soufre ou un azote ;
- un groupe méthylène substitué par un hétérocycle à 5 ou 6 chaînons, aromatique ou saturé, l'hétéroatome étant un oxygène, un azote ou un soufre, les atomes d'azote et de soufre pouvant être oxydés sous forme de N-oxyde ou de S-oxyde ;
lorsque R₄ est différent de H, pris ensemble R₃ et R₄ forment un cycle saturé à 5 ou 6 chaînons
R₅ et R₆ représentent indépendamment l'un de l'autre :
- un hydrogène,
- une chaîne hydrocarbonée saturée, linéaire ou ramifiée, comportant de 1 à 6 atomes de carbone, éventuellement substitué par un radical OH, OR₁₁, SH ou SR₁₁, COOH ou COOR₁₁, dans chacun de ces radicaux R₁₁ a la même signification que précédemment,
- un radical phényle ou benzyle, éventuellement substitué par :
* une chaîne alkyle, linéaire ou ramifiée, de 1 à 4 atomes de carbone ;
* 1 à 5 halogènes, notamment le fluor ou le brome ;
* un radical OH, OR₁₁ ou SR₁₁, R₁₁ ayant la même définition que précédemment ;
ou pris ensemble R₅ et R₆ forment un cycle saturé à 5 ou 6 chaînons R₇ représente
- un hydrogène ;
- un radical phényle ou benzyle éventuellement substitué par 1 à 5 halogènes, notamment le fluor ;
- un groupement de formule CR₁₂(Ru)C(O)OR₁₄ ;
- un groupement CR₁₂(R₁₃)OC(O)R₁₄;
- un groupement CR₁₂(R₁₃)OC(O)OR)₁₄;
R₁₂ et R₁₃ représentent indépendamment l'un de l'autre un hydrogène, un groupement alkyle, aryle, arylalkyle, cycloalkyle, cyclohétéroalkyle, hétéroalkyle, hétéroaryle ou hétéroaryalkyle ;
ou pris ensemble R₁₂ et R₁₃ peuvent constituer un cycloalkyle à 5 ou 6 chaînons ;
R₁₄ représente un groupement alkyle, aryle, arylalkyle, cycloalkyle, cyclohétéroalkyle, hétéroalkyle, hétéroaryle ou hétéroaryalkyle ;
ainsi que les sels d'addition desdits composés (I) avec des bases minérales ou organiques pharmaceutiquement acceptables et chacun de leurs isomères, en particulier leurs énantiomères optiques.

2. Composé selon la revendication 1, **caractérisé en ce que** le radical R₁ représente avantageusement un groupement (acyloxy)alkyl carbamate -C(O)-O-C(R₈)(R₉)-OC(O)-R₁₀, dans lequel R₈ et R₉ représentent indépendamment l'un de l'autre un hydrogène ou un groupement alkyle, et R₁₀ représente un groupement alkyle, en particulier un isopropyle.

3. Composé selon l'une quelconque des revendications précédentes, **caractérisé en ce que** le radical R₂ représente un radical alkyle ayant de 1 à 4 atomes de carbone substitué par un radical SR₁₁ ou S(O)R_{11,} R₁₁ ayant la même signification que dans la revendication 1, en particulier R₁₁ représente une chaîne hydrocarbonée saturée linéaire ou ramifiée de 1 à 4 atomes de carbone et plus particulièrement un groupe méthyle.

4. Composé selon l'une quelconque des revendications précédentes, **caractérisé en ce que** le radical R₄ représente un atome d'hydrogène.

5. Composé selon la revendication 4, **caractérisé en ce que** le radical R₃ représente :
- un radical benzyle ou phényle,
- un radical méthylène substitué par un hétérocycle à 5 ou 6 atomes, aromatique ou saturé, possédant comme hétéroatome, un atome d'azote ou de soufre, éventuellement oxydé sous forme de N-oxyde ou de S-oxyde.

6. Composé selon l'une quelconque des revendications 1 à 3, **caractérisé en ce que** les radicaux R₄ et R₃ forment conjointement, avec l'atome de carbone qui les porte un cycloalkyle à 5 ou 6 chaînons, en particulier un cyclopentane ou un cyclohexane.

7. Composé selon l'une quelconque des revendications précédentes, **caractérisé en ce que** le radical R₅ représente un atome d'hydrogène.

8. Composé selon l'une quelconque des revendications précédentes, **caractérisé en ce que** le radical R₆ représente un atome d'hydrogène ou un radical alkyle ayant de 1 à 6 atomes de carbone, substitué par un radical OH, SH, COOH ou COOR₁₁, dans chacun de ces radicaux R₁₁ a la même signification que précédemment.

9. Composé selon l'une quelconque des revendications précédentes, **caractérisé en ce que** le radical R₇ représente un atome d'hydrogène, un radical phényle ou benzyle ou un radical alkyle ayant de 1 à 4 atomes de carbone.

10. Composé selon l'une quelconque des revendications précédentes, **caractérisé en ce qu'**il est choisi parmi les composés suivants:
Ester 1-(1-{2-[(1-éthoxycarbonyloxy-éthoxycarbonylméthyl)-carbamoyl]-3-phényl-propyldisulfanylméthyl}-3-méthylsulfanyl-propylcarbamoyloxy)-éthyl de l'acide isobutyrique.
Ester 1-{1-[2-(benzyloxycarbonylméthyl-carbamoyl)-3-phényl-propyldisulfanylméthyl] -3-méthylsulfanyl-propylcarbamoyloxy}-éthyl de l'acide isobutyrique
Ester 1-{1-[2-(carboxymétyl-carbamoyl)-3-phényl-propyldisulfanylméthyl]-3-méthylsulfanyl-propylcarbamoyloxy}-éthyl de l'acide isobutyrique
Ester 1-(1-{2-[(1-éthoxycarbonyloxy-éthoxycarbonylméthyl)-carbamoyl]-3-phényl-propyldisulfanylméthyl}-3-méthanesulfinyl-propylcarbamoloxy)-éthyl de l'acide isobutyrique
Ester 1-{1-[2-benzyloxycarbonylméthyl-carbamoyl)-3-phényl-propyldisulfanylméthyl]-3-méthanesulfinyl-propylcarbamoyloxy}-éthyl de l'acide isobutyrique
Ester 1-{1-2[2-(carboxyméthyl-carbamoyl)-3-phényl-propyldisulfanylméthyl]-3-méthanesulfinyl-propylcarbamoyloxy}-éthyl de l'acide isobutyrique
Acide 2-({1-[2-(1-Isobutyryloxy-ethoxycarbonylamino)-4-methanesulfinyl-butyldisulfanylmethyl]-cyclopentanecarbonyl}-amino)-succinique
Acide 2-({1-[2-(1-Isobutyryloxy-ethoxycarbonylamino)-4-methylsulfanyl-butyldisulfanylmethyl]-cyclopentanecarbonyl}-amino)-succinique
Ester benzylique de l'acide 2-({1-[2-(1-Isobutyryloxy-ethoxycarbonylamino)-4-methanesulfinyl-butyldisulfanylmethyl]-cyclopentanecarbonyl}-amino)-succinique
Ester benzylique de l'acide 2-({1-[2-(1-Isobutyryloxy-ethoxycarbonylamino)-4-methylsulfanyl-butyldisulfanylmethyl]-cyclopentanecarbonyl}-amino)-succinique

11. A titre de médicament, le composé de formule (I) selon l'une quelconque des revendications précédentes.

12. Composition pharmaceutique, **caractérisée en ce qu'**elle comprend au moins un composé de formule (1), selon l'une quelconque des revendications 1 à 10, et un excipient pharmaceutiquement approprié, en particulier un excipient approprié pour une administration par voie orale, nasale ou intraveineuse.

13. Composition pharmaceutique selon la revendication 12, **caractérisée en ce qu'**elle comprend en outre au moins un dérivé de cannabinoïdes, en particulier le Δ⁹-tetrahydrocannabinol, ou de la morphine ou l'un de ses dérivés ou, plus avantageusement, un dérivé du Gaba, en particulier la gabapentine ou la prégabaline.

14. Composition pharmaceutique selon les revendications 12 ou 13, **caractérisée en ce qu'**elle est destinée au traitement de la dépression, de la sclérose en plaque et des différents types de douleur, tels que la douleur aigue, la douleur inflammatoire, la douleur nociceptive la douleur neurogénique, la douleur neuropathique, la douleur psychogène, l'allodynie.

15. Composition pharmaceutique comprenant
i) au moins un composé de formule (I), selon l'une quelconque des revendications 1 à 10
ii) au moins un dérivé de cannabinoïdes et/ou
iii) la morphine ou l'un de ses dérivés, et/ou
iv) au moins un dérivé du Gaba, tel que la gabapentine ou la prégabaline, en tant que produits de combinaison pour une utilisation simultanée, séparée ou étalée dans le temps.

16. Composition selon la revendication 15, destinée au traitement de la dépression, des addictions et de la douleur, en particulier la douleur inflammatoire, la douleur nociceptive, la douleur neurogénique, la douleur neuropathique, la douleur psychogène et leurs caractéristiques telles que l'hyperalgie et l'allodynie.

## Claims

1. A compound having the following formula (I):
R₁NH-CH(R₂)-CH₂-S-S-CH₂-C(R₃)(R₄)-CONH-C-(R₅)(R₆)-COOR₇
wherein:
R₁ represents an (acyloxy)alkyl carbamate group
- (CO)-O-C(R₈)(R₉)-OC(O)-R₁₀, wherein
- R₈ and R₉ independently of each other represent a hydrogen atom, an alkyl, aryl, arylakyl, cycloalkyl, cycloheteroalkyl, heteroalkyl, heteroaryl or heteroarylalkyl group; or
- taken together, R₈ and R₉ may form a 5- or 6-membered cycloalkyl;
- R₁₀ represents an alkyl, aryl, arylalkyl, cycloalkyl, cycloheteroalkyl, heteroalkyl, heteroaryl or heteroarylalkyl group;
R₂ represents:
- a linear or branched, saturated hydrocarbon chain having 1 to 6 carbon atoms, optionally substituted with:
❖ an OH, OR₁₁, SH, SR₁₁ or S(O)R₁₁ radical, in each of these radicals, R₁₁ represents a linear or branched hydrocarbon chain with 1 to 4 carbon atoms, a phenyl radical or a benzyl radical,
❖ a phenyl or benzyl radical, optionally substituted with:
• 1 to 5 halogen atoms, notably fluorine,
• an OH, OR₁₁, SH, SR₁₁ or S(O)R₁₁ radical, R₁₁ having the same meaning as earlier,
- a methylene radical substituted with a 5 or 6 members, aromatic or saturated, heterocycle, the heteroatom being, a nitrogen or sulfur atom, optionally oxidized as an N-oxide or S-oxide,
when R₄ represents a hydrogen atom, R₃ represents:
- a phenyl or benzyl radical optionally substituted with:
❖ 1 to 5 halogen atoms;
❖ an SR₁₁, S(O)R₁₁ or OR₁₁ radical, R₁₁ having the same meaning as earlier;
❖ an amino group optionally mono- or disubstituted with a cyclic or linear aliphatic group having 1 to 6 carbon atoms;
- a 5- or 6-membered heteroaryl, the heteroatom being an oxygen, a sulfur or nitrogen atom;
- a methylene group substituted with an aromatic or saturated, 5- or 6-membered heterocycle, the heteroatom being an oxygen, nitrogen or sulfur atom, the nitrogen and sulfur atoms may be oxidized as an N-oxide or S-oxide;
when R₄ is different from H, R₃ and R₄ taken together form a saturated 5- or 6-membered ring;
R₅ and R₆ independently of each other represent:
- a hydrogen atom,
- a linear or branched saturated hydrocarbon chain, having from 1 to 6 carbon atoms, optionally substituted with an OH, OR₁₁, SH or SR₁₁, COOH or COOR₁₁ radical, in each of these radicals, R₁₁ has the same meaning as earlier,
- a phenyl or benzyl radical, optionally substituted with:
❖ a linear or branched alkyl chain with 1 to 4 carbon atoms;
❖ 1 to 5 halogens, notably fluorine or bromine;
❖ an OH, OR₁₁, SH or SR₁₁ radical, R₁₁ having the same definition as earlier;
or taken together R₅ and R₆ form a saturated 5- or 6-membered ring
R₇ represents
- a hydrogen atom;
- a phenyl or benzyl radical optionally substituted with 1 to 5 halogens, notably fluorine;
- a group of formula CR₁₂(R₁₃)C(O)OR₁₄;
- a group CR₁₂(R₁₃)OC(O)R₁₄;
- a group CR₁₂(R₁₃)OC(O)OR₁₄;
R₁₂ and R₁₃ independently of each other represent a hydrogen atom, an alkyl, aryl, arylalkyl, cycloalkyl, cycloheteroalkyl, heteroalkyl, heteroaryl or heteroarylalkyl group;
or taken together R₁₂ and R₁₃ may form a 5- or 6-membered cycloalkyl;
R₁₄ represents an alkyl, aryl, arylalkyl, cycloalkyl, cycloheteroalkyl, heteroalkyl, heteroaryl or heteroarylalkyl group;
as well as the addition salts of said compound (I) with pharmaceutically acceptable mineral or organic bases and each of their isomers, in particular their optical enantiomers.

2. The compound according to claim 1, **characterized in that** the radical R₁ advantageously represents an (acyloxy)alkyl carbamate group -(CO)-O-C(R₈)(R₉)-OC(O)-R₁₀, wherein R₈ and R₉ independently of each other represent a hydrogen atom or an alkyl group; and R₁₀ represents an alkyl group, in particular an isopropyl.

3. The compound according to any of the preceding claims, **characterized in that** the radical R₂ represents an alkyl radical having from 1 to 4 carbon atoms, substituted with an SR₁₁ or S(O)R₁₁ radical, R₁₁ having the same meaning as in claim 1, in particular R₁₁ represents a linear or branched saturated hydrocarbon chain with 1 to 4 carbon atoms and more particularly a methyl group.

4. The compound according to any of the preceding claims, **characterized in that** the radical R₄ represents a hydrogen atom.

5. The compound according to claim 4, **characterized in that** the radical R₃ represents:
- a benzyl or phenyl radical,
- a methylene radical substituted with either an aromatic or saturated 5- or 6-membered heterocycle, having as a heteroatom a nitrogen or sulfur atom, optionally oxidized as an N-oxide or S-oxide.

6. The compound according to any of claims 1 to 3, **characterized in that** the radicals R₄ and R₃ form together with the carbon which bears them, a 5- or 6-membered cycloalkyl, in particular a cyclopentane or a cyclohexane.

7. The compound according to any of the preceding claims, **characterized in that** the radical R₅ represents a hydrogen atom.

8. The compound according to any of the preceding claims, **characterized in that** the radical R₆ represents a hydrogen atom or an alkyl radical having from 1 to 6 carbon atoms, substituted with an OH, SH, COOH or COOR₁₁ radical, in each of these radicals, R₁₁ has the same meaning as earlier.

9. The compound according to any of the preceding claims, **characterized in that** the radical R₇ represents a hydrogen atom, a phenyl or benzyl radical or an alkyl radical having from 1 to 4 carbon atoms.

10. The compound according to any of the preceding claims, **characterized in that** it is selected from the following compounds:
1-(1-{2-[(1-ethoxycarbonyloxy-ethoxy-carbonylmethyl)-carbamoyl] -3 -phenyl- propyldisulfanylmethyl}-3-methylsulfanyl-propylcarbamoyloxy)-ethyl isobutyric acid ester,
1-{1-[2-(benzyloxycarbonylmethyl-carbamoyl)-3-phenylpropyl-disulfanylmethyl]-3-methylsulfanyl-propylcarbamoyloxy}-ethyl isobutyric acid ester,
1-{1-[2-(carboxymethyl-carbamoyl)-3-phenyl-propyl-disulfanylmethyl]-3-methylsulfanyl-propylcarbamoyloxy}-ethyl isobutyric acid ester,
1-(1-{2-[(1-ethoxycarbonyloxy-ethoxycarbonylmethyl)-carbamoyl]-3-phenyl-propyldisulfanylmethyl}-3-methane-sulfinyl-propylcarbamoloxy)-ethyl isobutyric acid ester,
1-{1-[2-benzyloxycarbonylmethyl-carbamoyl)-3-phenylpropyl-disulfanylmethyl]-3-methanesulfinyl-propylcarbamoyloxy}-ethyl isobutyric acid ester,
1-{1-2[2-(carboxymethyl-carbamoyl)-3-phenyl-propyl-disulfanylmethyl]-3-methanesulfinyl-propylcarbamoyl-oxy}-ethyl isobutyric acid ester,
2-({1-[2-(1-isobutyryloxy-ethoxycarbonylamino)-4-methane-sulfinyl-butyldisulfanylmethyl]-cyclopentanecarbonyl}-amino)-succinic acid,
2-({1-[2-(1-isobutyryloxy-ethoxycarbonylamino)-4-methyl-sulfanyl-butyldisulfanylmethyl]-cyclopentanecarbonyl}-amino)-succinic acid, Benzyl
2-({1-[2-(1-isobutyryloxy-ethoxycarbonylamino)-4-methanesulfinyl-butyldisulfanylmethyl]-cyclopentanecarbonyl}-amino)-succinic acid ester Benzyl
2-({1-[2-(1-isobutyryloxy-ethoxycarbonylamino)-4-methylsulfanyl-butyldisulfanylmethyl]-cyclopentanecarbonyl}-amino)-succinic acid ester.

11. As a drug, the compound formula (I) according to any of the preceding claims.

12. A pharmaceutical composition, **characterized in that** it comprises at least one compound of formula (I), according to any of claims 1 to 10, and a pharmaceutically appropriate excipient, in particular an appropriate excipient for administration via an oral, nasal or intravenous route.

13. The pharmaceutical composition according to claim 12, **characterized in that** it further comprises at least one derivative of cannabinoids, in particular Δ⁹-tetrahydrocannabinol, or morphine or one of its derivatives, or more advantageously a derivative of Gaba, in particular gabapentin or pregabalin.

14. The pharmaceutical composition according to claims 12 or 13, **characterized in that** it is intended for the treatment of depression, multiple sclerosis, and different types of pain, such as acute pain, inflammatory pain, nociceptive pain, neurogenic pain, neuropathic pain, psychogenic pain, allodynia.

15. A pharmaceutical composition comprising
i) at least one compound of formula (I), according to any of claims 1 to 10
ii) at least one derivative of cannabinoids and/or
iii) morphine or one of its derivatives, and/or
iv) at least one derivative of Gaba, such as gabapentin or pregabalin, as combination products for simultaneous, separate use or spread out in time.

16. The composition according to claim 15, intended for treating depression, addictions and pain, in particular inflammatory pain, nociceptive pain, neurogenic pain, neuropathic pain, psychogenic pain, and their characteristics such as hyperalgesia and allodynia.

## Patentansprüche

1. Verbindung gemäß folgender Formel (I):
R₁NH-CH(R₂)-CH₂-S-S-CH₂-C(R₃)(R₄)-CONH-C(R₅)(R₆)-COOR₇,
worin:
R1 eine (Acyloxy)alkylcarbamatgruppe -C(O)-O-C(R₈)(R₉)-OC(O)-R₁₀ darstellt, worin
- R₈ und R₉ unabhängig voneinander einen Wasserstoff, eine Alkyl-, Aryl-, Arylalkyl-, Cycloalkyl-, Cycloheteroalkyl-, Heteroalkyl-, Heteroaryl- oder Heteroarylalkylgruppe darstellen; oder
- R₈ und R₉ zusammen ein 5- oder 6-gliedriges Cycloalkyl bilden können;
- R₁₀ eine Alkyl-, Aryl-, Arylalkyl-, Cycloalkyl-, Cycloheteroalkyl-, Heteroalkyl-, Heteroaryl- oder Heteroarylalkylgruppe darstellt;
R₂ darstellt:
- eine lineare oder verzweigte gesättigte Kohlenwasserstoffkette mit 1 bis 6 Kohlenstoffatomen, die gegebenenfalls substituiert ist mit:
* einem OH-, OR₁₁-, SH-, SR₁₁- oder S(O)R₁₁-Rest, wobei in jedem dieser Reste R₁₁ eine lineare oder verzweigte Kohlenwasserstoffkette mit 1 bis 4 Kohlenstoffatomen, einen Phenylrest oder einen Benzylrest darstellt,
* einem Phenyl- oder Benzylrest, der gegebenenfalls substituiert ist mit:
• 1 bis 5 Halogenatomen, insbesondere Fluor,
• einem OH-, OR₁₁-, SH-, SR₁₁- oder S(O)R₁₁-Rest, wobei R₁₁ die gleiche Bedeutung hat wie oben,
- einen Methylenrest, der mit einem aromatischen oder gesättigten 5- oder 6-gliedrigen Heterozyklus substituiert ist, der als Heteroatom ein Stickstoff- oder Schwefelatom aufweist, das gegebenenfalls in Form eines N-Oxids oder S-Oxids oxidiert ist, wenn R₄ ein Wasserstoffatom darstellt, R₃ darstellt:
- einen Phenyl- oder Benzylrest, der gegebenenfalls substituiert ist mit:
* 1 bis 5 Halogenatomen;
* einem SR₁₁-, S(O)R₁₁- oder OR₁₁-Rest, wobei R₁₁ die gleiche Bedeutung hat wie oben;
* einer Aminogruppe, die gegebenenfalls durch eine zyklische oder lineare aliphatische Gruppe mit 1 bis 6 Kohlenstoffatomen mono- oder disubstituiert ist,
- ein 5- oder 6-gliedriges Heteroaryl, wobei das Heteroatom Sauerstoff, Schwefel oder Stickstoff ist;
- eine Methylengruppe, die mit einem aromatischen oder gesättigten 5- oder 6-gliedrigen Heterozyklus substituiert ist, wobei das Heteroatom Sauerstoff, Stickstoff oder Schwefel ist, wobei die Stickstoff- und Schwefelatome in Form eines N-Oxids oder S-Oxids oxidiert sein können; wenn R₄ kein H ist, R₃ und R₄ zusammen einen gesättigten 5- oder 6-gliedrigen Zyklus bilden;
R₅ und R₆ unabhängig voneinander darstellen:
- einen Wasserstoff,
- eine lineare oder verzweigte gesättigte Kohlenwasserstoffkette mit 1 bis 6 Kohlenstoffatomen, die gegebenenfalls mit einem OH-, OR₁₁-, SH-, SR₁₁-, COOH-oder COOR₁₁-Rest substituiert ist, wobei jeder dieser R₁₁-Reste die gleiche Bedeutung hat wie oben,
- einen Phenyl- oder Benzylrest, der gegebenenfalls substituiert ist mit:
* einer linearen oder verzweigten Alkylkette mit 1 bis 4 Kohlenstoffatomen;
* 1 bis 5 Halogenatomen, insbesondere Fluor oder Brom;
* einem OH-, OR₁₁- oder SR₁₁-Rest, wobei R₁₁ wie oben definiert ist;
oder R₅ und R₆ zusammen einen gesättigten 5- oder 6-gliedrigen Zyklus bilden;
R₇ darstellt:
- einen Wasserstoff;
- einen Phenyl- oder Benzylrest, der gegebenenfalls mit 1 bis 5 Halogenatomen, insbesondere Fluor, substituiert ist;
- eine Gruppe der Formel CR₁₂(R₁₃) C(O) OR₁₄;
- eine Gruppe der Formel CR₁₂(R₁₃)OC(O)R₁₄;
- eine Gruppe der Formel CR₁₂(R₁₃)OC(O)OR₁₄;
wobei R₁₂ und R₁₃ unabhängig voneinander einen Wasserstoff, eine Alkyl-, Aryl-, Arylalkyl-, Cycloalkyl-, Cycloheteroalkyl-, Heteroalkyl-, Heteroaryl- oder Heteroarylalkylgruppe darstellen;
oder R₁₂ und R₁₃ zusammen ein 5- oder 6-gliedriges Cycloalkyl bilden können;
R₁₄ eine Alkyl-, Aryl-, Arylalkyl-, Cycloalkyl-, Cycloheteroalkyl-, Heteroalkyl-, Heteroaryl- oder
Heteroarylalkylgruppe darstellt;
sowie die Additionssalze dieser Verbindungen (I) mit pharmazeutisch akzeptablen mineralischen oder organischen Basen und jede ihrer Isomere, insbesondere ihre optischen Enantiomere.

2. Verbindung gemäß Anspruch 1, **dadurch gekennzeichnet, dass** der Rest R₁ vorteilhafterweise eine (Acyloxy)alkylcarbamatgruppe
-C(O)-O-C(R₈)(R₉)-OC(O)-R₁₀ darstellt, worin R₈ und R₉ unabhängig voneinander einen Wasserstoff oder eine Alkylgruppe darstellen, und R₁₀ eine Alkylgruppe, insbesondere eine Isopropylgruppe, darstellt.

3. Verbindung gemäß irgendeinem der vorhergehenden Ansprüche, **dadurch gekennzeichnet, dass** der Rest R₂ einen Alkylrest mit 1 bis 4 Kohlenstoffatomen, der mit einem SR₁₁-oder S(O)R₁₁-Rest substituiert ist, darstellt, wobei R₁₁ die gleiche Bedeutung wie in Anspruch 1 hat und R₁₁ insbesondere eine lineare oder verzweigte gesättigte Kohlenwasserstoffkette mit 1 bis 4 Kohlenstoffatomen, insbesondere eine Methylgruppe, darstellt.

4. Verbindung gemäß irgendeinem der vorhergehenden Ansprüche, **dadurch gekennzeichnet, dass** der Rest R₄ ein Wasserstoffatom darstellt.

5. Verbindung gemäß Anspruch 4, **dadurch gekennzeichnet, dass** der Rest R₃ darstellt:
- einen Benzyl- oder Phenylrest,
- einen Methylenrest, der mit einem aromatischen oder gesättigten Heterozyklus mit 5 oder 6 Atomen substituiert ist, der als Heteroatom ein Stickstoff- oder Schwefelatom, gegebenenfalls in Form eines oxidierten N-Oxids oder S-Oxids, aufweist.

6. Verbindung gemäß irgendeinem der Ansprüche 1 bis 3, **dadurch gekennzeichnet, dass** die Reste R₄ und R₃ in Verbindung mit dem Kohlenstoffatom, das sie trägt, ein 5- oder 6-gliedriges Cycloalkyl, insbesondere ein Cyclopentan oder Cyclohexan, bilden.

7. Verbindung gemäß irgendeinem der vorhergehenden Ansprüche, **dadurch gekennzeichnet, dass** der Rest R₅ ein Wasserstoffatom darstellt.

8. Verbindung gemäß irgendeinem der vorhergehenden Ansprüche, **dadurch gekennzeichnet, dass** der Rest R₆ ein Wasserstoffatom oder einen Alkylrest mit 1 bis 6 Kohlenstoffatomen, der mit einem OH-, SH-, COOH- oder COOR₁₁-Rest substituiert ist, darstellt, wobei R₁₁ die gleiche Bedeutung wie oben hat.

9. Verbindung gemäß irgendeinem der vorhergehenden Ansprüche, **dadurch gekennzeichnet, dass** der Rest R₇ ein Wasserstoffatom, einen Phenyl- oder Benzylrest oder einen Alkylrest mit 1 bis 4 Kohlenstoffatomen darstellt.

10. Verbindung gemäß irgendeinem der vorhergehenden Ansprüche, **dadurch gekennzeichnet, dass** sie aus den folgenden Verbindungen ausgewählt ist:
1-(1-{2-[(1-ethoxycarbonyloxy-ethoxycarbonylmethyl)-carbamoyl]-3-phenyl-propyldisulfanylmethyl}-3-methylsulfanyl-propylcarbamoyloxy)-isobuttersäureethylester,
1-{1-[2-(benzyloxycarbonylmethyl-carbamoyl)-3-phenyl-propyldisulfanylmethyl]-3-methylsulfanyl-propylcarbamoyloxy}-isobuttersäureethylester,
1-{1-[2-(carboxymethyl-carbamoyl)-3-phenyl-propyldisulfanylmethyl]-3-methylsutfanyl-propylcarbamoyloxy}-isobuttersäureethylester,
1-(1-{2-[(1-ethoxycarbonyloxy-ethoxycarbonylmethyl)-carbamoyl]-3-phenyl-propyldisulfanylmethyl}-3-methansulfinyl-propylcarbamoyloxy)-isobuttersäureethylester,
1-{1-[2-(benzyloxycarbonylmethyl-carbamoyl)-3-phenyl-propyldisulfanylmethyl]-3-methansulfinyl-propylcarbamoyloxy)-isobuttersäureethylester,
1-{1-2[2-(carboxymethyl-carbamoyl)-3-phenyl-propyldisulfanylmethyl]-3-methansulfinyl-propylcarbamoyloxy}-isobuttersäureethylester,
2-({1-[2-(1-Isobutyryloxy-ethoxycarbonylamino)-4-methansulfinyl-butyldisulfanylmethyl]cyclopentancarbonyl}-amino)-bernsteinsäure,
2-({1-[2-(1-Isobutyryloxy-ethoxycarbonylamino)-4-methylsulfanyl-butyldisulfanylmethyl]-cyclopentancarbonyl}-amino)-bernsteinsäure,
2-({1-[2-(1-Isobutyryloxy-ethoxycarbonylamino)-4-methansulfinyl-butyldisulfanylmethyl]-cyclopentancarbonyl}-amino)-bernsteinsäurebenzylester,
2-({1-[2-(1-Isobutyryloxy-ethoxycarbonylamino)-4-methylsulfanyl-butyldisulfanylmethyl]-cyclopentancarbonyl}-amino)-bernsteinsäurebenzylester.

11. Verbindung der Formel (I) gemäß irgendeinem der vorhergehenden Ansprüche als Medikament.

12. Pharmazeutische Zusammensetzung, **dadurch gekennzeichnet, dass** sie wenigstens eine Verbindung der Formel (I) gemäß irgendeinem der Ansprüche 1 bis 10 und einen pharmazeutisch geeigneten Träger, insbesondere einen Träger, der für eine Verabreichung auf oralem, nasalem oder intravenösem Weg geeignet ist, umfasst.

13. Pharmazeutische Zusammensetzung gemäß Anspruch 12, **dadurch gekennzeichnet, dass** sie außerdem wenigstens ein Cannabinoid-Derivat, insbesondere Δ⁹-Tetrahydrocannabinol, oder Morphin oder eines seiner Derivate oder, vorteilhafterweise, ein Gaba-Derivat, insbesondere Gabapentin oder Pregabalin, enthält.

14. Pharmazeutische Zusammensetzung gemäß Anspruch 12 oder 13, **dadurch gekennzeichnet, dass** sie zur Behandlung von Depression, Multipler Sklerose und verschiedener Schmerztypen, wie akutem Schmerz, Entzündungsschmerz, nocizeptivem Schmerz, neurogenem Schmerz, neuropathischem Schmerz, psychogenem Schmerz und Allodynie, bestimmt ist.

15. Pharmazeutische Zusammensetzung, umfassend:
i) wenigstens eine Zusammensetzung der Formel (I) gemäß irgendeinem der Ansprüche 1 bis 10,
ii) wenigstens ein Cannabinoid-Derivat und/oder
iii) Morphin oder eines seiner Derivate und/oder
iv) wenigstens ein Gaba-Derivat, wie Gabapentin oder Pregabalin,
als Kombinationspräparat zur gleichzeitigen, zeitlich getrennten oder sich über einen Zeitraum erstreckenden Verwendung.

16. Zusammensetzung gemäß Anspruch 15 zur Behandlung von Depression, Suchterkrankungen und Schmerzzuständen, insbesondere Entzündungsschmerz, nocizeptivem Schmerz, neurogenem Schmerz, neuropathischem Schmerz, psychogenem Schmerz und deren Ausprägungen, wie Hyperalgesie und Allodynie.
